(19) Europäisches Patentamt
European Patent Office
Office européen des brevets

(11) **EP 2 596 364 B1**

(12) **EUROPEAN PATENT SPECIFICATION**

(45) Date of publication and mention of the grant of the patent:
**16.12.2015 Bulletin 2015/51**

(51) Int Cl.:
**G01N 33/574** (2006.01)

(21) Application number: **11748601.9**

(22) Date of filing: **18.07.2011**

(86) International application number:
**PCT/EP2011/062226**

(87) International publication number:
**WO 2012/010546 (26.01.2012 Gazette 2012/04)**

(54) **BLOOD PLASMA BIOMARKERS FOR BEVACIZUMAB COMBINATION THERAPIES FOR TREATMENT OF PANCREATIC CANCER**

BLUTPLASMABIOMARKER FÜR BEVACIZUMAB-KOMBINATIONSTHERAPIEN ZUR BEHANDLUNG VON PANKREASKARZINOM

BIOMARQUEURS DU PLASMA SANGUIN POUR DES POLYTHÉRAPIES À BASE DE BEVACIZUMAB, DANS LE TRAITEMENT DU CANCER DU PANCRÉAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.07.2010 EP 10170004**

(43) Date of publication of application:
**29.05.2013 Bulletin 2013/22**

(73) Proprietor: **F.Hoffmann-La Roche AG**
**4070 Basel (CH)**

(72) Inventors:
• **DELMAR, Paul**
  **CH-4057 Basel (CH)**
• **FOERNZLER, Dorothee**
  **CH-5600 Lenzburg (CH)**
• **KRAUSE, Friedemann**
  **82377 Penzberg (DE)**
• **SCHERER, Stefan**
  **California 94080 (US)**

(74) Representative: **Vossius & Partner**
**Patentanwälte Rechtsanwälte mbB**
**Siebertstrasse 3**
**81675 München (DE)**

(56) References cited:
**WO-A1-2010/075420    US-A1- 2005 170 444**

• **S. X. YANG ET AL: "Gene Expression Profile and Angiogenic Markers Correlate with Response to Neoadjuvant Bevacizumab Followed by Bevacizumab plus Chemotherapy in Breast Cancer", CLINICAL CANCER RESEARCH, vol. 14, no. 18, 15 September 2008 (2008-09-15), pages 5893-5899, XP055000181, ISSN: 1078-0432, DOI: 10.1158/1078-0432.CCR-07-4762**
• **D. G. DUDA ET AL: "Plasma Soluble VEGFR-1 Is a Potential Dual Biomarker of Response and Toxicity for Bevacizumab with Chemoradiation in Locally Advanced Rectal Cancer", THE ONCOLOGIST, vol. 15, no. 6, 1 June 2010 (2010-06-01), pages 577-583, XP055010526, ISSN: 1083-7159, DOI: 10.1634/theoncologist. 2010-0029**
• **ANDREW H KO ET AL: "A phase II study evaluating bevacizumab in combination with fixed-dose rate gemcitabine and low-dose cisplatin for metastatic pancreatic cancer: is an anti-VEGF strategy still applicable?", INVESTIGATIONAL NEW DRUGS ; THE JOURNAL OF NEW ANTICANCER AGENTS, KLUWER ACADEMIC PUBLISHERS, BO, vol. 26, no. 5, 1 April 2008 (2008-04-01), pages 463-471, XP019602934, ISSN: 1573-0646**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HEDY L KINDLER ET AL: "Phase II trial of bevacizumab plus gemcitabine in patients with advanced pancreatic cancer", JOURNAL OF CLINICAL ONCOLOGY, AMERICAN SOCIETY OF CLINICAL ONCOLOGY, US, vol. 23, no. 31, 1 January 2005 (2005-01-01), pages 8033-8040, XP008132250, ISSN: 0732-183X, DOI: 10.1200/JCO.2005.01.9661 [retrieved on 2005-11-01]
- ANASTASIOS J. KARAYIANNAKIS ET AL: "Serum vascular endothelial growth factor levels in pancreatic cancer patients correlate with advanced and metastatic disease and poor prognosis", CANCER LETTERS, vol. 194, no. 1, 1 May 2003 (2003-05-01), pages 119-124, XP55013431, ISSN: 0304-3835, DOI: 10.1016/S0304-3835(03)00047-8
- VAN CUTSEM ERIC ET AL: "Phase III trial of bevacizumab in combination with gemcitabine and erlotinib in patients with metastatic pancreatic cancer.", JOURNAL OF CLINICAL ONCOLOGY : OFFICIAL JOURNAL OF THE AMERICAN SOCIETY OF CLINICAL ONCOLOGY 1 MAY 2009 LNKD- PUBMED:19307500, vol. 27, no. 13, 1 May 2009 (2009-05-01), pages 2231-2237, XP002664669, ISSN: 1527-7755
- LONGO R ET AL: "Pancreatic cancer: From molecular signature to target therapy", CRITICAL REVIEWS IN ONCOLOGY / HEMATOLOGY, ELSEVIER SCIENCE IRELAND LTD., LIMERICK, IE, vol. 68, no. 3, 1 December 2008 (2008-12-01), pages 197-211, XP025780310, ISSN: 1040-8428, DOI: 10.1016/J.CRITREVONC.2008.03.003 [retrieved on 2008-04-23]

SEQUENCE LISTING

<110> F. Hoffmann-La Roche AG et al.

<120> Blood plasma biomarkers for Bevacizumab combination therapies for treatment of pancreatic cancer

<130> S1945 PCT S3

<150> EP 10 17 0004.5
<151> 2010-07-19

<160> 3

<170> PatentIn version 3.3

<210> 1
<211> 232
<212> PRT
<213> Homo sapiens

<400> 1

```
Met Asn Phe Leu Leu Ser Trp Val His Trp Ser Leu Ala Leu Leu Leu
1               5               10                  15

Tyr Leu His His Ala Lys Trp Ser Gln Ala Ala Pro Met Ala Glu Gly
        20              25                  30

Gly Gly Gln Asn His His Glu Val Val Lys Phe Met Asp Val Tyr Gln
        35              40                  45

Arg Ser Tyr Cys His Pro Ile Glu Thr Leu Val Asp Ile Phe Gln Glu
    50              55                  60

Tyr Pro Asp Glu Ile Glu Tyr Ile Phe Lys Pro Ser Cys Val Pro Leu
65              70                  75                  80

Met Arg Cys Gly Gly Cys Cys Asn Asp Glu Gly Leu Glu Cys Val Pro
            85              90                  95

Thr Glu Glu Ser Asn Ile Thr Met Gln Ile Met Arg Ile Lys Pro His
            100             105                 110

Gln Gly Gln His Ile Gly Glu Met Ser Phe Leu Gln His Asn Lys Cys
        115             120                 125

Glu Cys Arg Pro Lys Lys Asp Arg Ala Arg Gln Glu Lys Lys Ser Val
    130             135                 140

Arg Gly Lys Gly Lys Gly Gln Lys Arg Lys Arg Lys Lys Ser Arg Tyr
145             150                 155                 160

Lys Ser Trp Ser Val Tyr Val Gly Ala Arg Cys Cys Leu Met Pro Trp
            165             170                 175

Ser Leu Pro Gly Pro His Pro Cys Gly Pro Cys Ser Glu Arg Arg Lys
            180             185                 190

His Leu Phe Val Gln Asp Pro Gln Thr Cys Lys Cys Ser Cys Lys Asn
        195             200                 205

Thr Asp Ser Arg Cys Lys Ala Arg Gln Leu Glu Leu Asn Glu Arg Thr
    210             215                 220

Cys Arg Cys Asp Lys Pro Arg Arg
225             230
```

<210> 2
<211> 1356

<212> PRT
<213> Homo sapiens

<400> 2

```
Met Gln Ser Lys Val Leu Leu Ala Val Ala Leu Trp Leu Cys Val Glu
1               5                   10                  15

Thr Arg Ala Ala Ser Val Gly Leu Pro Ser Val Ser Leu Asp Leu Pro
            20                  25                  30

Arg Leu Ser Ile Gln Lys Asp Ile Leu Thr Ile Lys Ala Asn Thr Thr
            35                  40                  45

Leu Gln Ile Thr Cys Arg Gly Gln Arg Asp Leu Asp Trp Leu Trp Pro
        50                  55                  60

Asn Asn Gln Ser Gly Ser Glu Gln Arg Val Glu Val Thr Glu Cys Ser
65                  70                  75                  80

Asp Gly Leu Phe Cys Lys Thr Leu Thr Ile Pro Lys Val Ile Gly Asn
                85                  90                  95

Asp Thr Gly Ala Tyr Lys Cys Phe Tyr Arg Glu Thr Asp Leu Ala Ser
                100                 105                 110

Val Ile Tyr Val Tyr Val Gln Asp Tyr Arg Ser Pro Phe Ile Ala Ser
            115                 120                 125

Val Ser Asp Gln His Gly Val Val Tyr Ile Thr Glu Asn Lys Asn Lys
        130                 135                 140

Thr Val Val Ile Pro Cys Leu Gly Ser Ile Ser Asn Leu Asn Val Ser
145                 150                 155                 160
```

```
Leu Cys Ala Arg Tyr Pro Glu Lys Arg Phe Val Pro Asp Gly Asn Arg
              165                 170             175

Ile Ser Trp Asp Ser Lys Lys Gly Phe Thr Ile Pro Ser Tyr Met Ile
              180                 185             190

Ser Tyr Ala Gly Met Val Phe Cys Glu Ala Lys Ile Asn Asp Glu Ser
              195             200             205

Tyr Gln Ser Ile Met Tyr Ile Val Val Val Gly Tyr Arg Ile Tyr
      210                 215             220

Asp Val Val Leu Ser Pro Ser His Gly Ile Glu Leu Ser Val Gly Glu
225             230                 235                 240

Lys Leu Val Leu Asn Cys Thr Ala Arg Thr Glu Leu Asn Val Gly Ile
              245                 250                 255

Asp Phe Asn Trp Glu Tyr Pro Ser Ser Lys His Gln His Lys Lys Leu
              260             265             270

Val Asn Arg Asp Leu Lys Thr Gln Ser Gly Ser Glu Met Lys Lys Phe
              275             280             285

Leu Ser Thr Leu Thr Ile Asp Gly Val Thr Arg Ser Asp Gln Gly Leu
      290             295             300

Tyr Thr Cys Ala Ala Ser Ser Gly Leu Met Thr Lys Lys Asn Ser Thr
305             310             315                 320

Phe Val Arg Val His Glu Lys Pro Phe Val Ala Phe Gly Ser Gly Met
              325             330             335

Glu Ser Leu Val Glu Ala Thr Val Gly Glu Arg Val Arg Ile Pro Ala
          340             345             350

Lys Tyr Leu Gly Tyr Pro Pro Pro Glu Ile Lys Trp Tyr Lys Asn Gly
      355             360             365

Ile Pro Leu Glu Ser Asn His Thr Ile Lys Ala Gly His Val Leu Thr
      370             375             380

Ile Met Glu Val Ser Glu Arg Asp Thr Gly Asn Tyr Thr Val Ile Leu
385             390             395             400

Thr Asn Pro Ile Ser Lys Glu Lys Gln Ser His Val Val Ser Leu Val
              405             410             415

Val Tyr Val Pro Pro Gln Ile Gly Glu Lys Ser Leu Ile Ser Pro Val
```

                    420                        425                          430

Asp Ser Tyr Gln Tyr Gly Thr Thr Gln Thr Leu Thr Cys Thr Val Tyr
        435             440             445

Ala Ile Pro Pro Pro His His Ile His Trp Tyr Trp Gln Leu Glu Glu
    450             455             460

Glu Cys Ala Asn Glu Pro Ser Gln Ala Val Ser Val Thr Asn Pro Tyr
465             470             475             480

Pro Cys Glu Glu Trp Arg Ser Val Glu Asp Phe Gln Gly Gly Asn Lys
            485             490             495

Ile Glu Val Asn Lys Asn Gln Phe Ala Leu Ile Glu Gly Lys Asn Lys
        500             505             510

Thr Val Ser Thr Leu Val Ile Gln Ala Ala Asn Val Ser Ala Leu Tyr
        515             520             525

Lys Cys Glu Ala Val Asn Lys Val Gly Arg Gly Glu Arg Val Ile Ser
    530             535             540

Phe His Val Thr Arg Gly Pro Glu Ile Thr Leu Gln Pro Asp Met Gln
545             550             555             560

Pro Thr Glu Gln Glu Ser Val Ser Leu Trp Cys Thr Ala Asp Arg Ser
            565             570             575

Thr Phe Glu Asn Leu Thr Trp Tyr Lys Leu Gly Pro Gln Pro Leu Pro
        580             585             590

Ile His Val Gly Glu Leu Pro Thr Pro Val Cys Lys Asn Leu Asp Thr
        595             600             605

Leu Trp Lys Leu Asn Ala Thr Met Phe Ser Asn Ser Thr Asn Asp Ile
    610             615             620

Leu Ile Met Glu Leu Lys Asn Ala Ser Leu Gln Asp Gln Gly Asp Tyr
625             630             635             640

Val Cys Leu Ala Gln Asp Arg Lys Thr Lys Lys Arg His Cys Val Val
            645             650             655

Arg Gln Leu Thr Val Leu Glu Arg Val Ala Pro Thr Ile Thr Gly Asn
        660             665             670

Leu Glu Asn Gln Thr Thr Ser Ile Gly Glu Ser Ile Glu Val Ser Cys
        675             680             685

Thr Ala Ser Gly Asn Pro Pro Pro Gln Ile Met Trp Phe Lys Asp Asn
    690             695             700

Glu Thr Leu Val Glu Asp Ser Gly Ile Val Leu Lys Asp Gly Asn Arg
705             710             715             720

Asn Leu Thr Ile Arg Arg Val Arg Lys Glu Asp Glu Gly Leu Tyr Thr
            725             730             735

Cys Gln Ala Cys Ser Val Leu Gly Cys Ala Lys Val Glu Ala Phe Phe
            740             745             750

Ile Ile Glu Gly Ala Gln Glu Lys Thr Asn Leu Glu Ile Ile Ile Leu
        755             760             765

Val Gly Thr Ala Val Ile Ala Met Phe Phe Trp Leu Leu Leu Val Ile
        770             775             780

Ile Leu Arg Thr Val Lys Arg Ala Asn Gly Gly Glu Leu Lys Thr Gly
785             790             795             800

Tyr Leu Ser Ile Val Met Asp Pro Asp Glu Leu Pro Leu Asp Glu His
            805             810             815

Cys Glu Arg Leu Pro Tyr Asp Ala Ser Lys Trp Glu Phe Pro Arg Asp
            820             825             830

Arg Leu Lys Leu Gly Lys Pro Leu Gly Arg Gly Ala Phe Gly Gln Val
        835             840             845

Ile Glu Ala Asp Ala Phe Gly Ile Asp Lys Thr Ala Thr Cys Arg Thr
    850             855             860

Val Ala Val Lys Met Leu Lys Glu Gly Ala Thr His Ser Glu His Arg
865             870             875             880

Ala Leu Met Ser Glu Leu Lys Ile Leu Ile His Ile Gly His His Leu
            885             890             895

Asn Val Val Asn Leu Leu Gly Ala Cys Thr Lys Pro Gly Gly Pro Leu
            900             905             910

Met Val Ile Val Glu Phe Cys Lys Phe Gly Asn Leu Ser Thr Tyr Leu
        915             920             925

Arg Ser Lys Arg Asn Glu Phe Val Pro Tyr Lys Thr Lys Gly Ala Arg
    930             935             940

Phe Arg Gln Gly Lys Asp Tyr Val Gly Ala Ile Pro Val Asp Leu Lys
945                 950                 955                 960

Arg Arg Leu Asp Ser Ile Thr Ser Ser Gln Ser Ser Ala Ser Ser Gly
                965                 970                 975

Phe Val Glu Glu Lys Ser Leu Ser Asp Val Glu Glu Glu Glu Ala Pro
                980                 985                 990

Glu Asp Leu Tyr Lys Asp Phe Leu  Thr Leu Glu His Leu  Ile Cys Tyr
        995                 1000                1005

Ser Phe  Gln Val Ala Lys Gly  Met Glu Phe Leu Ala  Ser Arg Lys
    1010                1015                1020

Cys Ile  His Arg Asp Leu Ala  Ala Arg Asn Ile Leu  Leu Ser Glu
    1025                1030                1035

Lys Asn  Val Val Lys Ile Cys  Asp Phe Gly Leu Ala  Arg Asp Ile
    1040                1045                1050

Tyr Lys  Asp Pro Asp Tyr Val  Arg Lys Gly Asp Ala  Arg Leu Pro
    1055                1060                1065

Leu Lys  Trp Met Ala Pro Glu  Thr Ile Phe Asp Arg  Val Tyr Thr
    1070                1075                1080

Ile Gln  Ser Asp Val Trp Ser  Phe Gly Val Leu Leu  Trp Glu Ile
    1085                1090                1095

Phe Ser  Leu Gly Ala Ser Pro  Tyr Pro Gly Val Lys  Ile Asp Glu
    1100                1105                1110

Glu Phe  Cys Arg Arg Leu Lys  Glu Gly Thr Arg Met  Arg Ala Pro
    1115                1120                1125

Asp Tyr  Thr Thr Pro Glu Met  Tyr Gln Thr Met Leu  Asp Cys Trp
    1130                1135                1140

His Gly  Glu Pro Ser Gln Arg  Pro Thr Phe Ser Glu  Leu Val Glu
    1145                1150                1155

His Leu  Gly Asn Leu Leu Gln  Ala Asn Ala Gln Gln  Asp Gly Lys
    1160                1165                1170

Asp Tyr  Ile Val Leu Pro Ile  Ser Glu Thr Leu Ser  Met Glu Glu
    1175                1180                1185

```
Asp Ser Gly Leu Ser Leu Pro Thr Ser Pro Val Ser Cys Met Glu
    1190                1195            1200

Glu Glu Glu Val Cys Asp Pro Lys Phe His Tyr Asp Asn Thr Ala
    1205                1210            1215

Gly Ile Ser Gln Tyr Leu Gln Asn Ser Lys Arg Lys Ser Arg Pro
    1220                1225            1230

Val Ser Val Lys Thr Phe Glu Asp Ile Pro Leu Glu Glu Pro Glu
    1235                1240            1245

Val Lys Val Ile Pro Asp Asp Asn Gln Thr Asp Ser Gly Met Val
    1250                1255            1260

Leu Ala Ser Glu Glu Leu Lys Thr Leu Glu Asp Arg Thr Lys Leu
    1265                1270            1275

Ser Pro Ser Phe Gly Gly Met Val Pro Ser Lys Ser Arg Glu Ser
    1280                1285            1290

Val Ala Ser Glu Gly Ser Asn Gln Thr Ser Gly Tyr Gln Ser Gly
    1295                1300            1305

Tyr His Ser Asp Asp Thr Asp Thr Thr Val Tyr Ser Ser Glu Glu
    1310                1315            1320

Ala Glu Leu Leu Lys Leu Ile Glu Ile Gly Val Gln Thr Gly Ser
    1325                1330            1335

Thr Ala Gln Ile Leu Gln Pro Asp Ser Gly Thr Thr Leu Ser Ser
    1340                1345            1350

Pro Pro Val
    1355
```

<210> 3
<211> 221
<212> PRT
<213> Homo sapiens

<400> 3

```
        Met Pro Val Met Arg Leu Phe Pro Cys Phe Leu Gln Leu Leu Ala Gly
        1               5               10              15

        Leu Ala Leu Pro Ala Val Pro Pro Gln Gln Trp Ala Leu Ser Ala Gly
                    20              25              30

        Asn Gly Ser Ser Glu Val Glu Val Val Pro Phe Gln Glu Val Trp Gly
                35              40              45

        Arg Ser Tyr Cys Arg Ala Leu Glu Arg Leu Val Asp Val Val Ser Glu
            50              55              60

        Tyr Pro Ser Glu Val Glu His Met Phe Ser Pro Ser Cys Val Ser Leu
        65              70              75              80

        Leu Arg Cys Thr Gly Cys Cys Gly Asp Glu Asn Leu His Cys Val Pro
                    85              90              95

        Val Glu Thr Ala Asn Val Thr Met Gln Leu Leu Lys Ile Arg Ser Gly
                100             105             110

        Asp Arg Pro Ser Tyr Val Glu Leu Thr Phe Ser Gln His Val Arg Cys
                115             120             125

        Glu Cys Arg His Ser Pro Gly Arg Gln Ser Pro Asp Met Pro Gly Asp
            130             135             140

        Phe Arg Ala Asp Ala Pro Ser Phe Leu Pro Pro Arg Arg Ser Leu Pro
        145             150             155             160

        Met Leu Phe Arg Met Glu Trp Gly Cys Ala Leu Thr Gly Ser Gln Ser
                    165             170             175

        Ala Val Trp Pro Ser Ser Pro Val Pro Glu Glu Ile Pro Arg Met His
                    180             185             190

        Pro Gly Arg Asn Gly Lys Lys Gln Gln Arg Lys Pro Leu Arg Glu Lys
                195             200             205

        Met Lys Pro Glu Arg Cys Gly Asp Ala Val Pro Arg Arg
            210             215             220
```

**Description**

The present disclosure provides methods for improving the treatment effect of a chemotherapy regimen of a patient suffering from pancreatic cancer, in particular metastatic pancreatic cancer by adding bevacizumab (Avastin®) to a chemotherapy regimen by determining the expression level, in particular the blood plasma expression level, of one or more of VEGFA, VEGFR2 and PLGF relative to control levels of patients diagnosed with pancreatic cancer, in particular metastatic pancreatic cancer. In particular, the present disclosure provides methods of improving the treatment effect, wherein the treatment effect is the overall survival and/or progression-free survival of the patient. The present disclosure further provides for methods for assessing the sensitivity or responsiveness of a patient to bevacizumab (Avastin®) in combination with a chemotherapy regimen, by determining the expression level, in particular the blood plasma expression level, of one or more of VEGFA, VEGFR2 and PLGF relative to control levels in patients diagnosed with pancreatic cancer, in particular metastatic pancreatic cancer.

Accordingly, the present disclosure relates to the identification and selection of biomarkers of pancreatic cancer, in particular metastatic pancreatic cancer, that correlate with sensitivity or responsiveness to angiogenesis inhibitors, e.g., bevacizumab (Avastin®), in combination with chemotherapeutic regimens, such as gemcitabine-erlotinib (GE) therapy. In this respect, the disclosure relates to the use of (a) blood plasma specific expression profile(s) of one or more of VEGFA, VEGFR2 and PLGF relative to controls established in patients diagnosed with pancreatic cancer, in particular metastatic pancreatic cancer, to identify patients sensitive or responsive to the addition of angiogenesis inhibitors, e.g., bevacizumab (Avastin®), to standard chemotherapies. The disclosure further relates to methods for improving the treatment effect, in particular, the overall survival and/or progression-free survival of a patient suffering from pancreatic cancer, in particular metastatic pancreatic cancer, by the addition of angiogenesis inhibitors, e.g., bevacizumab (Avastin®), to standard chemotherapies, e.g., gemcitabine-erlotinib (GE) therapy, by determining (a) blood plasma specific expression level(s) of one or more of VEGFA, VEGFR2 and PLGF relative to control(s) in patients diagnosed with pancreatic cancer, in particular metastatic pancreatic cancer. The disclosure further provides for kits and compositions for identification of patients sensitive or responsive to angiogenesis inhibitors, in particular, bevacizumab (Avastin®), determined and defined in accordance with the methods of the present disclosure.

Angiogenesis is necessary for cancer development, regulating not only primary tumor size and growth but also impacting invasive and metastatic potential. Accordingly, the mechanisms mediating angiogenic processes have been investigated as potential targets for directed anti-cancer therapies. Early in the study of angiogenic modulators, the vascular endothelial growth factor (VEGF) signalling pathway was discovered to preferentially regulate angiogenic activity in multiple cancer types. This factor signals through VEGF Receptor 2 (VEGFR-2), the major VEGF signalling receptor that mediates angiogenesis. Multiple therapeutics have been developed to modulate this pathway at various points. These therapies include, among others, bevacizumab, sunitinib, sorafenib and vatalanib. Although the use of angiogenic inhibitors in the clinic has shown success, not all patients respond or fail to fully respond to angiogenesis inhibitor therapy. The mechanism(s) underlying such incomplete response is unknown. Therefore, there is an increasing need for the identification of patient subgroups sensitive or responsive to anti-angiogenic cancer therapy.

While a number of angiogenesis inhibitors are known, the most prominent angiogenesis inhibitor is bevacizumab (Avastin®). Bevacizumab is a recombinant humanized monoclonal IgG1 antibody that specifically binds and blocks the biological effects of VEGF (vascular endothelial growth factor). VEGF is a key driver of tumor angiogenesis - an essential process required for tumor growth and metastasis, *i.e.,* the dissemination of the tumor to other parts of the body. Avastin® is approved in Europe for the treatment of the advanced stages of four common types of cancer: colorectal cancer, breast cancer, non-small cell lung cancer (NSCLC) and kidney cancer, which collectively cause over 2.5 million deaths each year. In the United States, Avastin® was the first anti-angiogenesis therapy approved by the FDA, and it is now approved for the treatment of five tumor types: colorectal cancer, non-small cell lung cancer, breast cancer, brain (glioblastoma) and kidney (renal cell carcinoma). Over half a million patients have been treated with Avastin so far, and a comprehensive clinical program with over 450 clinical trials is investigating the further use of Avastin in the treatment of multiple cancer types (including colorectal, breast, non-small cell lung, brain, gastric, ovarian and prostate) in different settings (e.g., advanced or early stage disease). Importantly, Avastin® has shown promise as a co-therapeutic, demonstrating efficacy when combined with a broad range of chemotherapies and other anti-cancer treatments. Phase-III studies have been published demonstrating the beneficial effects of combining bevacizumab with standard chemotherapeutic regimens (see, *e.g.,* Saltz et al., 2008, J. Clin. Oncol, 26:2013-2019; Yang et al., 2008, Clin. Cancer Res., 14:5893-5899; Hurwitz et al., 2004, N. Engl. J. Med., 350:2335-2342). However, as in previous studies of angiogenic inhibitors, some of these phase-III studies have shown that a portion of patients experience incomplete response to the addition of bevacizumab (Avastin®) to their chemotherapeutic regimens.

Accordingly, there is a need for methods of determining those patients that respond to or are likely to respond to combination therapies comprising angiogenesis inhibitors, in particular, bevacizumab (Avastin®). Thus, the technical problem underlying the present invention is the provision of methods and means for the identification of (a) patient(s) suffering from or prone to suffer from pancreatic cancer, in particular metastatic pancreatic cancer, who may benefit from the addition of angiogenesis inhibitors, in particular, bevacizumab (Avastin®), to chemotherapeutic therapies, e.g., gemcitabine-erlotinib (GE) therapy.

The technical problem is solved by provision of the embodiments characterized in the claims. Accordingly, the invention relates to the embodiments as defined in the claims. Thus, the invention relates to the following items:

1. An in vitro method for the identification of a patient responsive to or sensitive to the addition of bevacizumab treatment to a chemotherapy regimen, said method comprising determining the protein expression level of one or more of VEGFA, VEGFR2 and PLGF in a blood plasma sample from a patient suspected to suffer from or being prone to suffer from pancreatic cancer, whereby an increased expression level of one or more of VEGFA, VEGFR2 and PLGF relative to control expression levels determined in patients suffering from pancreatic cancer is indicative of a sensitivity of the patient to the addition of bevacizumab to said chemotherapy regimen, wherein the pancreatic cancer is metastatic pancreatic cancer and the chemotherapy regimen comprises gemcitabine and erlotinib.

2. An in vitro method of predicting the response to or sensitivity to the addition of bevacizumab to a chemotherapy regimen of a patient suspected to suffer from, suffering from or prone to suffer from pancreatic cancer comprising determining the protein expression level of one or more of VEGFA, VEGFR2 and PLGF in a patient blood plasma sample, wherein the pancreatic cancer is metastatic pancreatic cancer and the chemotherapy regimen comprises gemcitabine and erlotinib.

3. The method of item 1, wherein the treatment effect is progression-free survival.

4. The method of item 1, wherein the treatment effect is overall survival.

5. The method of items 2 to 4, wherein the protein expression level determined is of VEGFA or PLGF.

6. The method of items 2 to 4, wherein the protein expression level determined is of VEGFA or VEGFR2.

7. The method of items 1 to 4, wherein protein expression level determined is a combined expression level of VEGFA and VEGFR2.

8. The method of items 1 to 4, wherein the protein expression level determined is a combined expression level of VEGFA and PLGF.

9. The method of items 1 to 4, wherein the protein expression level determined is a combined expression level of VEGFA, VEGFR2 and PLGF.

10. The method of items 1 to 9, wherein said expression level is detected by an immunoassay method.

11. The method of item 10, wherein said immunoassay method is ELISA.

12. The method of items 1 to 11, wherein said patient is being co-treated with one or more anti-cancer therapies.

13. The method of item 12, wherein said anti-cancer therapy is radiation.

14. The method of items 1 to 13, wherein said sample is obtained before neoadjuvant or adjuvant therapy or after neoadjuvant or adjuvant therapy.

In addition, the present disclosure provides a method for improving the treatment effect of a chemotherapy regimen of a patient suffering from pancreatic cancer by adding bevacizumab to said chemotherapy regimen, said method comprising:

(a) determining the protein expression level of one or more of VEGFA, VEGFR2 and PLGF in a patient sample; and
(b) administering bevacizumab in combination with a chemotherapy regimen to the patient having an increased expression level of one or more of VEGFA, VEGFR2 and PLGF relative to control expression levels determined in patients diagnosed with pancreatic cancer.

The present disclosure relates to a method for improving the treatment effect of a chemotherapy regimen of a patient suffering from pancreatic cancer by adding bevacizumab to the chemotherapy regimen, said method comprising:

(a) obtaining a sample from said patient;

(b) determining the protein expression level of one or more of VEGFA, VEGFR2 and PLGF; and

(c) administering bevacizumab in combination with a chemotherapy regimen to the patient having an increased expression level of one or more of VEGFA, VEGFR2 and PLGF relative to control expression levels determined in patients diagnosed with pancreatic cancer.

The present disclosure relates to a method for improving the overall survival of a patient suffering from pancreatic cancer by adding bevacizumab to a chemotherapy regimen, said method comprising:

(a) determining the protein expression level of one or more of VEGFA, VEGFR2 and PLGF in a patient sample; and

(b) administering bevacizumab in combination with the chemotherapy regimen to the patient having an increased expression level of one or more of VEGFA, VEGFR2 and PLGF relative to control expression levels determined in patients diagnosed with pancreatic cancer.

The present disclosure relates to a method for improving the overall survival of a patient suffering from pancreatic cancer by adding bevacizumab to a chemotherapy regimen, said method comprising:

(a) obtaining a sample from said patient;

(b) determining the protein expression level of one or more of VEGFA, VEGFR2 and PLGF; and

(c) administering bevacizumab in combination with the chemotherapy regimen to the patient having an increased expression level of one or more of VEGFA, VEGFR2 and PLGF relative to control expression levels determined in patients diagnosed with pancreatic cancer.

The present disclosure relates to a method for improving the overall survival of a patient suffering from metastatic pancreatic cancer by adding bevacizumab to a chemotherapy regimen, said method comprising:

(a) determining the protein expression level of one or more of VEGFA, VEGFR2 and PLGF in a patient sample; and

(b) administering bevacizumab in combination with the chemotherapy regimen to the patient having an increased expression level of one or more of VEGFA, VEGFR2 and PLGF relative to control expression levels determined in patients diagnosed with metastatic pancreatic cancer,

wherein the chemotherapy regimen comprises gemcitabine-erlotinib therapy.

The present disclosure relates to a method for improving the overall survival of a patient suffering from metastatic pancreatic cancer by adding bevacizumab to a chemotherapy regimen, said method comprising:

(a) obtaining a sample from said patient;

(b) determining the protein expression level of one or more of VEGFA, VEGFR2 and PLGF; and

(c) administering bevacizumab in combination with the chemotherapy regimen to the patient having an increased expression level of one or more of VEGFA, VEGFR2 and PLGF relative to control expression levels determined in patients diagnosed with metastatic pancreatic cancer,

wherein the chemotherapy regimen comprises gemcitabine-erlotinib therapy.

The present disclsosure relates to a method for improving the progression free survival of a patient suffering from pancreatic cancer by adding bevacizumab to a chemotherapy regimen, said method comprising:

(a) determining the protein expression level of one or more of VEGFA, VEGFR2 and PLGF in a patient sample; and

(b) administering bevacizumab in combination with the chemotherapy regimen to the patient having an increased expression level of one or more of VEGFA, VEGFR2 and PLGF relative to control expression levels determined in patients diagnosed with pancreatic cancer.

The present disclosure relates to a method for improving the progression free survival of a patient suffering from pancreatic cancer by adding bevacizumab to a chemotherapy regimen, said method comprising:

(a) obtaining a sample from said patient;

(b) determining the protein expression level of one or more of VEGFA, VEGFR2 and PLGF; and

(c) administering bevacizumab in combination with the chemotherapy regimen to the patient having an increased expression level of one or more of VEGFA, VEGFR2 and PLGF relative to control expression levels determined in patients diagnosed with pancreatic cancer.

The present disclosure relates to a method for improving the progression free survival of a patient suffering from metastatic pancreatic cancer by adding bevacizumab to a chemotherapy regimen, said method comprising:

(a) determining the protein expression level of one or more of VEGFA, VEGFR2 and PLGF in a patient sample; and
(b) administering bevacizumab in combination with the chemotherapy regimen to the patient having an increased expression level of one or more of VEGFA, VEGFR2 and PLGF relative to control expression levels determined in patients diagnosed with metastatic pancreatic cancer,

wherein the chemotherapy regimen comprises gemcitabine-erlotinib therapy.

The present disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of one or more of VEGFA, VEGFR2 and PLGF; and
(c) administering bevacizumab in combination with the chemotherapy regimen to the patient having an increased expression level of one or more of VEGFA, VEGFR2 and PLGF relative to control expression levels determined in patients diagnosed with metastatic pancreatic cancer,

wherein the chemotherapy regimen comprises gemcitabine-erlotinib therapy.

The present disclosure relates to a method for improving the overall survival of a patient suffering from pancreatic cancer by adding bevacizumab to a chemotherapy regimen, said method comprising:

(a) determining the protein expression level of VEGFA or VEGFR2 in a patient sample; and
(b) administering bevacizumab in combination with the chemotherapy regimen to the patient having an increased expression level of VEGFA or VEGFR2 relative to control expression levels determined in patients diagnosed with pancreatic cancer.

The present disclosure relates to a method for improving the overall survival of a patient suffering from pancreatic cancer by adding bevacizumab to a chemotherapy regimen, said method comprising:

(a) obtaining a sample from said patient;
(b) determining the protein expression level of VEGFA or VEGFR2; and
(c) administering bevacizumab in combination with the chemotherapy regimen to the patient having an increased expression level VEGFA or VEGFR2 relative to control expression levels determined in patients diagnosed with pancreatic cancer.

The present disclosure relates to a method for improving the overall survival of a patient suffering from metastatic pancreatic cancer by adding bevacizumab to a chemotherapy regimen, said method comprising:

(a) determining the protein expression level of VEGFA or VEGFR2 in a patient sample; and
(b) administering bevacizumab in combination with the chemotherapy regimen to the patient having an increased expression level of VEGFA or VEGFR2 relative to control expression levels determined in patients diagnosed with metastatic pancreatic cancer,

wherein the chemotherapy regimen comprises gemcitabine-erlotinib therapy.

The present disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA or VEGFR2; and
(c) administering bevacizumab in combination with the chemotherapy regimen to the patient having an increased expression level of VEGFA or VEGFR2 relative to control expression levels determined in patients diagnosed with metastatic pancreatic cancer,
wherein the chemotherapy regimen comprises gemcitabine-erlotinib therapy.

The present disclosure relates to a method for improving the progression free survival of a patient suffering from pancreatic cancer by adding bevacizumab to a chemotherapy regimen, said method comprising:

(a) determining the protein expression level of VEGFA or PLGF in a patient sample; and
(b) administering bevacizumab in combination with the chemotherapy regimen to the patient having an increased expression level of VEGFA or PLGF relative to control expression levels determined in patients diagnosed with pancreatic cancer.

The present disclosure relates to a method for improving the progression free survival of a patient suffering from pancreatic cancer by adding bevacizumab to a chemotherapy regimen, said method comprising:

(a) obtaining a sample from said patient;
(b) determining the protein expression level of VEGFA or PLGF; and
(c) administering bevacizumab in combination with the chemotherapy regimen to the patient having an increased expression level of VEGFA or PLGF relative to control expression levels determined in patients diagnosed with pancreatic cancer.

The present disclosure relates to a method for improving the progression free survival of a patient suffering from metastatic pancreatic cancer by adding bevacizumab to a chemotherapy regimen, said method comprising:

(a) determining the protein expression level of VEGFA or PLGF in a patient sample; and
(b) administering bevacizumab in combination with the chemotherapy regimen to the patient having an increased expression level of VEGFA or PLGF relative to control expression levels determined in patients diagnosed with metastatic pancreatic cancer,
wherein the chemotherapy regimen comprises gemcitabine-erlotinib therapy.

The present disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA or PLGF; and
(c) administering bevacizumab in combination with the chemotherapy regimen to the patient having an increased expression level of VEGFA or PLGF relative to control expression levels determined in patients diagnosed with metastatic pancreatic cancer, wherein the chemotherapy regimen comprises gemcitabine-erlotinib therapy.

The disclosure provides a method for improving the overall survival of a patient suffering from pancreatic cancer by adding bevacizumab to a chemotherapy regimen, said method comprising:

(a) determining the protein expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with pancreatic cancer.

The pancreatic cancer may be metastatic pancreatic cancer.
Accordingly, the disclosure relates to a method for improving the overall survival of a patient suffering from pancreatic cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with pancreatic cancer.

The pancreatic cancer may be metastatic pancreatic cancer.
The disclosure, therefore, relates to a method for improving the overall survival of a patient suffering from metastatic pancreatic cancer by adding bevacizumab to a chemotherapy regimen, said method comprising:

(a) determining the protein expression level of VEGFA, VEGFR2 and PLGF in a patient sample; and
(b) administering bevacizumab in combination the chemotherapy regimen to the patient having an increased combined expression level of VEGFA, VEGFR2 and PLGF relative to a control combined expression level determined in patients diagnosed with metastatic pancreatic cancer,

wherein the chemotherapy regimen comprises gemcitabine-erlotinib therapy.

The disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with the chemotherapy regimen to the patient having an increased combined expression level of VEGFA, VEGFR2 and PLGF relative to a control combined expression level determined in patients diagnosed with metastatic pancreatic cancer,
wherein the chemotherapy regimen comprises gemcitabine-erlotinib therapy.

The disclosure provides a method for improving the progression free survival of a patient suffering from pancreatic cancer by adding bevacizumab to a chemotherapy regimen, said method comprising:

(a) determining the protein expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with pancreatic cancer.

The pancreatic cancer may be metastatic pancreatic cancer.

Accordingly, the disclosure relates to a method for improving the progression free survival of a patient suffering from pancreatic cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with pancreatic cancer.

The pancreatic cancer may be metastatic pancreatic cancer.

The disclosure, therefore, relates to a method for improving the progression free survival of a patient suffering from metastatic pancreatic cancer by adding bevacizumab to a chemotherapy regimen, said method comprising:

(a) determining the protein expression level of VEGFA, VEGFR2 and PLGF in a patient sample; and
(b) administering bevacizumab in combination the chemotherapy regimen to the patient having an increased combined expression level of VEGFA, VEGFR2 and PLGF relative to a control combined expression level determined in patients diagnosed with metastatic pancreatic cancer,
wherein the chemotherapy regimen comprises gemcitabine-erlotinib therapy.

The disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with the chemotherapy regimen to the patient having an increased combined expression level of VEGFA, VEGFR2 and PLGF relative to a control combined expression level determined in patients diagnosed with metastatic pancreatic cancer,

wherein the chemotherapy regimen comprises gemcitabine-erlotinib therapy.

The disclosure provides a method for improving the overall survival of a patient suffering from metastatic pancreatic cancer by adding bevacizumab to a chemotherapy regimen, said method comprising:

(a) determining the protein expression level of VEGFA and VEGFR2 in a patient sample; and
(b) administering bevacizumab in combination with a chemotherapy regimen to the patient having an increased combined expression level of VEGFA and VEGFR2 relative to a control combined expression level determined in patients diagnosed with metastatic pancreatic cancer.

Accordingly, the disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA and VEGFR2; and
(c) administering bevacizumab in combination with a chemotherapy regimen to the patient having an increased combined expression level of VEGFA and VEGFR2 relative to a control combined expression level determined in patients diagnosed with metastatic pancreatic cancer.

The disclosure, therefore, relates to a method for improving the overall survival of a patient suffering from metastatic pancreatic cancer by adding bevacizumab to a chemotherapy regimen, said method comprising:

(a) determining the protein expression level of VEGFA and VEGFR2 in a patient sample; and
(b) administering bevacizumab in combination the chemotherapy regimen to the patient having an increased combined expression level of VEGFA and VEGFR2 relative to a control combined expression level determined in patients diagnosed with metastatic pancreatic cancer,
wherein the chemotherapy regimen comprises gemcitabine-erlotinib therapy.

The disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA and VEGFR2; and
(c) administering bevacizumab in combination with the chemotherapy regimen to the patient having an increased combined expression level of VEGFA and VEGFR2 relative to a control combined expression level determined in patients diagnosed with metastatic pancreatic cancer,

wherein the chemotherapy regimen comprises gemcitabine-erlotinib therapy.
The disclosure provides a method for improving the progression free survival of a patient suffering from metastatic pancreatic cancer by adding bevacizumab to a chemotherapy regimen, said method comprising:

(a) determining the protein expression level of VEGFA and VEGFR2 in a patient sample; and
(b) administering bevacizumab in combination with the chemotherapy regimen to the patient having an increased combined expression level of VEGFA and VEGFR2 relative to a control combined expression level determined in patients diagnosed with metastatic pancreatic cancer.

Accordingly, the disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA and VEGFR2; and
(c) administering bevacizumab in combination with the chemotherapy regimen to the patient having an increased combined expression level of VEGFA and VEGFR2 relative to a control combined expression level determined in patients diagnosed with metastatic pancreatic cancer.

The disclosure, therefore relates to a method for improving the progression free survival of a patient suffering from metastatic pancreatic cancer by adding bevacizumab to a chemotherapy regimen, said method comprising:

(a) determining the protein expression level of VEGFA and VEGFR2 in a patient sample; and
(b) administering bevacizumab in combination with the chemotherapy regimen to the patient having an increased combined expression level of VEGFA and VEGFR2 relative to a control combined expression level determined in patients diagnosed with metastatic pancreatic cancer,
wherein the chemotherapy regimen comprises gemcitabine-erlotinib therapy.

The disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA and VEGFR2; and

(c) administering bevacizumab in combination with the chemotherapy regimen to the patient having an increased combined expression level of VEGFA and VEGFR2 relative to a control combined expression level determined in patients diagnosed with metastatic pancreatic cancer,

wherein the chemotherapy regimen comprises gemcitabine-erlotinib therapy.

The present disclosure provides a method for improving the overall survival of a patient suffering from metastatic pancreatic cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with metastatic pancreatic cancer.

Accordingly, the present disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with metastatic pancreatic cancer.

The disclosure, therefore, relates to a method for improving the overall survival of a patient suffering from metastatic pancreatic cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with metastatic pancreatic cancer,

wherein the chemotherapy regimen comprises gemcitabine-erlotinib therapy.

Accordingly, the present disclosure relates to a method for improving the overall survival of a patient suffering from metastatic pancreatic cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with metastatic pancreatic cancer,
wherein the chemotherapy regimen comprises gemcitabine-erlotinib therapy.

The present disclosure provides a method for improving the progression-free survival of a patient suffering from metastatic pancreatic cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with metastatic pancreatic cancer.

Accordingly, the present disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with metastatic pancreatic cancer.

The disclosure, therefore, relates to a method for improving the progression free survival of a patient suffering from metastatic pancreatic cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with metastatic pancreatic cancer,
wherein the chemotherapy regimen comprises gemcitabine-erlotinib therapy.

Accordingly, the present disclosure relates to a method for improving the progression free survival of a patient suffering from metastatic pancreatic cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with metastatic pancreatic cancer,

wherein the chemotherapy regimen comprises gemcitabine-erlotinib therapy.

The present disclosure provides an in vitro method for the identification of a patient responsive to or sensitive to the addition of bevacizumab treatment to a chemotherapy regimen, said method comprising determining the protein expression level of one or more of VEGFA, VEGFR2 and PLGF in a sample from a patient suffering from, suspected to suffer from or being prone to suffer from pancreatic cancer, in particular metastatic pancreatic cancer, whereby an increased expression level of one or more of VEGFA, VEGFR2 and PLGF relative to control expression levels determined in patients suffering from pancreatic cancer, in particular metastatic pancreatic cancer, is indicative of a sensitivity of the patient to the addition of bevacizumab to said chemotherapy regimen. The chemotherapy regimen may comprise gemcitabine-erlotinib therapy.

Accordingly, the present disclosure relates to an in vitro method for the identification of a patient responsive to or sensitive to the addition of bevacizumab treatment to a chemotherapy regimen, said method comprising:

(a) obtaining a sample from a patient suffering from, suspected to suffer from or being prone to suffer from pancreatic cancer, in particular metastatic pancreatic cancer; and
(b) determining the protein expression level of one or more of VEGFA, VEGFR2 and PLGF;
whereby an increased expression level of one or more of VEGFA, VEGFR2 and PLGF relative to control expression levels determined in patients suffering from pancreatic cancer, in particular metastatic pancreatic cancer, is indicative of a sensitivity of the patient to the addition of bevacizumab to said chemotherapy regimen. The chemotherapy regimen may comprise gemcitabine-erlotinib therapy.

The present disclosure provides an in vitro method for the identification of a patient that is responsive to or sensitive to the addition of bevacizumab treatment to a chemotherapy regimen, said method comprising determining the protein expression level of one or more of VEGFA, VEGFR2 and PLGF in a sample from a patient suffering from, suspected to suffer from or being prone to suffer from metastatic pancreatic cancer, whereby an increased combined expression increased level of VEGFA and VEGFR2 or VEGFA and PLGF or VEGFA, VEGFR2 and PLGF relative to a control combined expression level determined in patients suffering from metastatic pancreatic cancer is indicative of a sensitivity of the patient to the addition of bevacizumab to said chemotherapy regimen. The chemotherapy regimen may comprise gemcitabine-erlotinib therapy.

Accordingly, the present invention solves the identified technical problem in that it was surprisingly shown that the blood plasma specific expression levels of one or more of VEGFA, VEGFR2 and PLGF in a given patient, relative to control levels determined in patients diagnosed with pancreatic cancer, in particular, metastatic pancreatic, correlate with treatment effect in those patients administered an angiogenesis inhibitor in combination with a chemotherapy regimen. Specifically, variations in the protein expression levels of VEGFA, VEGFR2 and/or PLGF were surprisingly identified as markers/predictors for the improved overall survival and/or progression-free survival of metastatic pancreatic cancer patients in response to the addition of bevacizumab (Avastin®) to the chemotherapy regimen of gemcitabine-erlotinib. Patients exhibiting a response or sensitivity to the addition of bevacizumab (Avastin®) to chemotherapy regimens were identified to have an increased protein expression level of one or more VEGFA, VEGFR2 and PLGF relative to control expression levels established in samples obtained from patients diagnosed with pancreatic cancer, in particular, metastatic pancreatic cancer. The terms "marker" and "predictor" can be used interchangeably and refer to the expression levels of one or more of VEGFA, VEGFR2 and PLGF as described herein. The invention also encompasses the use of the terms "marker" and "predictor" to refer to a combination of any two or more of the blood plasma expression levels of VEGFA, VEGFR2 and PLGF.

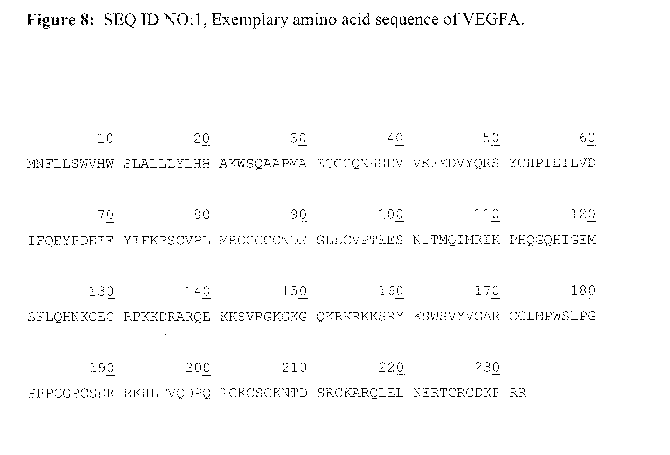

In the context of the present invention, "VEGFA" refers to vascular endothelial growth factor protein A, exemplified by SEQ ID NO:1, shown in FIGURE 8 (Swiss Prot Accession Number P15692, Gene ID (NCBI): 7422). The term "VEGFA" encompasses the protein having the amino acid sequence of SEQ ID NO:1 as well as homologues and isoforms thereof. The term "VEGFA" also encompasses the known isoforms, e.g., splice isoforms, of VEGFA, e.g., $VEGF_{111}$, $VEGF_{121}$, $VEGF_{145}$, $VEGF_{165}$, $VEGF_{189}$ and $VEGF_{206}$, as well as variants, homologues and isoforms thereof, including the 110- amino acid human vascular endothelial cell growth factor generated by plasmin cleavage of $VEGF_{165}$ as described in Ferrara Mol. Biol. Cell 21:687 (2010) and Leung et al. Science 246:1306 (1989), and Houck et al. Mol. Endocrin. 5:1806 (1991). In a particular embodiment of the present invention, "VEGFA" refers to $VEGF_{121}$ and/or $VEGF_{110}$. In a particular embodiment of the present invention, "VEGFA" refers to $VEGF_{111}$. In the context of the disclosure, the term "VEGFA" also encompasses proteins having at least 85%, at least 90% or at least 95% homology to the amino acid sequence of SEQ ID NO:1, or to the amino acid sequences of the variants and/or homologues thereof, as well as fragments of the sequences, provided that the variant proteins (including isoforms), homologous proteins and/or fragments are recognized by one or more VEGFA specific antibodies, such as antibody clone 3C5 and 26503, which are available from Bender RELIATech and R&D Systems, respectively and A4.6.1 as described in Kim et al., Growth Factors 7(1): 53-64 (1992). In the context of the invention, the term "isoform" of VEGF or VEGF-A refers to both splice isoforms and forms generated by enzymatic cleavage (e.g., plasmin).

In one embodiment, "VEGFA" refers to unmodified VEGF. In the context of the present invention "unmodified" VEGF relates to the unmodified amino acid sequence of VEGF, its isoforms and its cleavage products. Unmodified VEGF can e.g. be produced synthetically or preferably recombinantly in prokaryotic expression systems, e.g. in E. coli. Unmodified VEGF does e.g. not carry a posttranslational modification, like a glycosylation. In the context of the disclosure, the term "unmodified VEGF-A" also encompasses variants and/or homologues thereof, as well as fragments of VEGF-A, provided that the variant proteins (including isoforms), homologous proteins and/or fragments are recognized by an unmodified VEGF-A specific antibodies, such as antibody clone 3C5, which is available from RELIATech GmbH, Wolfenbüttel, Germany.

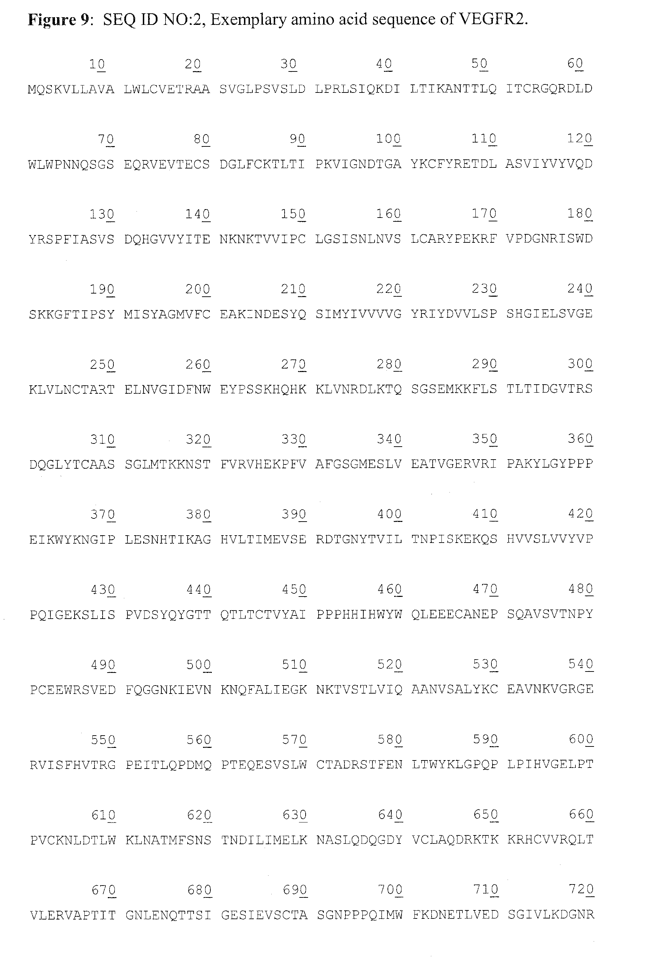

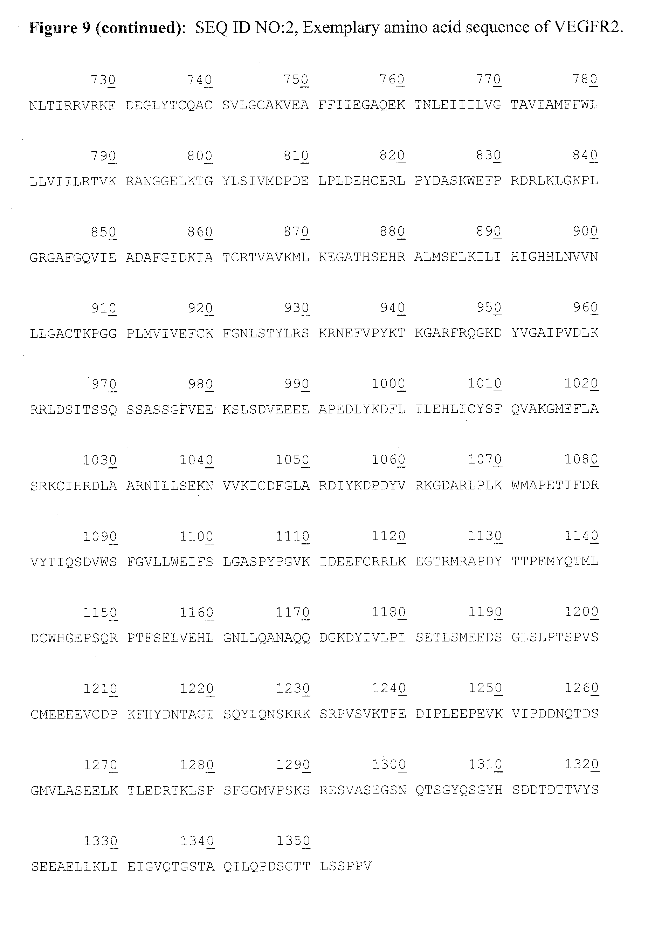

In the context of the present invention, "VEGFR2" refers to vascular endothelial growth factor receptor 2, exemplified by SEQ ID NO:2, shown in FIGURE 9 (Swiss Prot Accession Number P35968, Gene ID (NCBI): 3791). The term "VEGFR2" encompasses the protein having the amino acid sequence of SEQ ID NO:2 as well as homologues and isoforms thereof. In the context of the disclosure, the term "VEGFR2" also encompasses proteins having at least 85%, at least 90% or at least 95% homology to the amino acid sequence of SEQ ID NO:2, or to the amino acid sequences of the variants and/or homologues thereof, as well as fragments of the sequences, provided that the variant proteins (including isoforms), homologous proteins and/or fragments, are recognized by one or more VEGFR2 specific antibodies, such as antibody clone 89115 and 89109, which are available from R&D Systems.

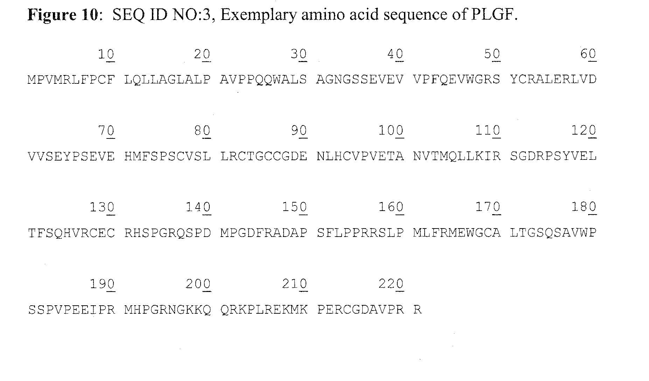

In the context of the present invention, "PLGF" refers to placental growth factor exemplified by SEQ ID NO:3, shown in FIGURE 10 (Swiss Prot Accession Number P49763, Gene ID (NCBI): 5228). The term "PLGF" encompasses the protein having the amino acid sequence of SEQ ID NO:3 as well as homologues and isoforms thereof. In the context of the disclosure, the term "PLGF" also encompasses proteins having at least 85%, at least 90% or at least 95% homology to the amino acid sequence of SEQ ID NO:3, or to the amino acid sequences of the variants and/or homologues thereof, as well as fragments of the sequences, provided that the variant proteins (including isoforms), homologous proteins and/or fragments are recognized by one or more PLGF specific antibodies, such as antibody clone 2D6D5 and 6A11D2, which are available from Roche Diagnostics GmbH.

Accordingly, the present disclosure encompasses the determination of expression levels of proteins including, but not limited to, the amino acid sequences as described herein. In this context the disclosure encompasses the detection of homologues, variants and isoforms of one or more of VEGFA, VEGFR2 and PLGF; said isoforms or variants may, inter alia, comprise allelic variants or splice variants. Also envisaged is the detection of proteins that are homologous to one or more of VEGFA, VEGFR2 and PLGF as herein described, or a fragment thereof, e.g., having at least 60%, 70%, 80%, 90%, 95%, 96%, 97%, 98% or 99% sequence identity to the amino acid sequence of SEQ ID NO:1, SEQ ID NO:2 or SEQ ID NO:3 or a fragment thereof. Alternatively or additionally, the present disclosure encompasses detection of the expression levels of proteins encoded by nucleic acid sequences, or fragments thereof, that are at least at least 60%, 70%, 80%, 90%, 95%, 96%, 97%, 98% or 99% identical to a nucleic acid sequence encoding SEQ ID NO:1, SEQ ID NO:2 or SEQ ID NO:3 or a fragment, variant or isoform thereof. In this context, the term "variant" means that the VEGFA, VEGFR2 and/or PLGF amino acid sequence, or the nucleic acid sequence encoding said amino acid sequence, differs from the distinct sequences identified by SEQ ID NOs:1, SEQ ID NO:2 or SEQ ID NO:3 and/or available under the above-identified Swiss Prot Accession numbers, by mutations, e.g., deletion, additions, substitutions, inversions etc. In addition, the term "homologue" references molecules having at least 60%, more preferably at least 80% and most preferably at least 90% sequence identity to one or more of the polypeptides as shown in SEQ ID NOs:1, SEQ ID NO:2 or SEQ ID NO:3, or (a) fragment(s) thereof.

In order to determine whether an amino acid or nucleic acid sequence has a certain degree of identity to an amino acid or nucleic acid sequence as herein described, the skilled person can use means and methods well known in the art, e.g. alignments, either manually or by using computer programs known in the art or described herein.

In accordance with the present disclosure, the term "identical" or "percent identity" in the context of two or more or amino acid or nucleic acid sequences, refers to two or more sequences or subsequences that are the same, or that have a specified percentage of amino acid residues or nucleotides that are the same (e.g., 60% or 65% identity, preferably, 70-95% identity, more preferably at least 95% identity with the amino acid sequences of, *e.g.,* SEQ ID NO:1, SEQ ID NO:2 or SEQ ID NO:3), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, *i.e.,* gaps, in its calculation, this can be corrected manually to avoid an overestimation of the % identity. CLUSTALW, however, does take sequence gaps into account in its identity calculations. Also available to those having skill in this art are the BLAST (Basic Local Alignment Search Tool) and BLAST 2.0 algorithms (Altschul, 1997, Nucl. Acids Res. 25:3389-3402; Altschul, 1993 J. Mol. Evol. 36:290-300; Altschul, 1990, J. Mol. Biol. 215:403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff (1989) PNAS 89:10915) uses alignments (B) of 50, expectation (E) of 10, M=5, N=4, and a comparison of both strands.

BLAST algorithms, as discussed above, produce alignments of both amino and nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST may be used to search for identical or related molecules in protein or nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\% \text{ sequence identity } \times \% \text{ maximum BLAST score}}{100}$$

and takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, 1994, Nucl. Acids Res. 2:4673-4680) or FASTDB (Brutlag, 1990, Comp. App. Biosci. 6:237-245), as is known in the art.

In the context of the herein described disclosure, the expression levels, in particular protein expression levels, of VEGFA, VEGFR2 and/or PLGF, may be considered separately, as individual markers, or in groups of two or more, as an expression profile or marker panel. In the context of the herein described disclosure an expression profile or marker panel wherein the expression profiles of two or more markers may be considered together may also be referred to as a combined expression level. For example, the expression levels of two or more markers may be added together and compared to a similarly determined control combined expression level. Therefore, the methods of the disclosure encompass determination of an expression profile, including a combined expression level, based on the expression level of one or more of the markers.

In the context of the herein described invention, and in accordance with the appended illustrative example, for consideration of VEGFA, VEGFR2 or PLGF separately, the following values were used as the corresponding high or low expression value of the marker: High VEGFA ($\geq$152.9 pg/ml), Low VEGFA (<152.9 pg/ml), High VEGFR2 ($\geq$9.9 ng/ml), Low VEGFR2 (<9.9 ng/ml). These levels were determined by the median of available samples as per pre-determined statistical analysis plan. Additionally, optimized levels constituting the cut-off value between high and low expression of a particular marker may be determined by varying the cut-off until the subset of patients above and below the cut-off satisfy a relevant statistical optimality criterion. For example, optimal cut-point may be chosen to maximize the differences in treatment Hazard Ratio between the subset above and below, or to maximize treatment effect in one sub-group, or any other relevant statistical criterion. In accordance with the herein described invention, and in accordance with the appended illustrative example, the optimized expression values for PLGF considered separately were High PLGF ($\geq$ 36.5 pg/ml), and Low PLGF (<36.5 pg/ml). This level was determined as 42nd percentile of available data. This level was determined in order to increase the statistical difference in treatment effect between high and low level. The skilled person will, however, understand that the expression level of the particular marker and, therefore, what constitutes a high or low expression level may vary by patient and by patient population. Accordingly, the skilled person will understand that when using detections methods other than those described in the appended illustrative example and studying patients and patient populations other than those described in the appended illustrative example, what the skilled person considers a high and/or low expression level for a particular biomarker may vary from the values herein described. Given the methods herein described, the skilled person can determine what constitutes a high and/or low level of expression of a particular biomarker.

As the skilled person will appreciate there are many ways to use the measurements of two or more markers in order to improve the diagnostic question under investigation. In a quite simple, but nonetheless often effective approach, a positive result is assumed if a sample is positive for at least one of the markers investigated.

However, a combination of markers may also be evaluated. The values measured for markers of a marker panel (or a combined expression level), e.g. for VEGFA and VEGFR2 or VEGFA and PLGF or VEGFA, VEGFR2 and PLGF, may be mathematically combined and the combined value may be correlated to the underlying diagnostic question. Marker values may be combined by any appropriate state of the art mathematical method. Well-known mathematical methods for correlating a marker combination to a disease or to a treatment effect employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem selecting an appropriate method to evaluate a marker combination of the present invention. The method used in correlating marker combinations in accordance with the invention herein disclosed with, for example improved overall survival, progression free survival, responsiveness or sensitivity to addition of bevacizumab to chemotherapeutic agents/chemotherapy regimen and/or the prediction of a response to or sensitivity to bevacizumab (in addition to one or more chemotherapeutic agents/chemotherapy regimen) is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

Accordingly, the disclosure herein disclosed relates to the use of an optimized multivariate cut-off for the underlying combination of biological markers and to discriminate state A from state B, e.g. patients responsive to or sensitive to the addition of bevacizumab to a chemotherapy regimen from patients that are poor responders to the addition of bevacizumab therapy to a chemotherapy regimen. In this type of analysis the markers are no longer independent but form a marker panel or a combined expression level.

The present disclosure, therefore, relates to method for improving the treatment effect of a chemotherapy regimen of patients suffering from pancreatic cancer, in particular metastatic pancreatic cancer, by adding bevacizumab to a chemotherapy regimen by determining the expression levels two or more of VEGFA, PLGF and VEGFR2, by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

The present disclosure, accordingly, relates to a method for improving the treatment effect of a chemotherapy regimen of patients suffering from cancer, in particular metastatic pancreatic cancer, by adding bevacizumab to a chemotherapy regimen by determining the expression levels of VEGFA and VEGFR2, and by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

The present disclosure also relates to a method for improving the treatment effect of a chemotherapy regimen of patients suffering from cancer, in particular metastatic pancreatic cancer, by adding bevacizumab to a chemotherapy regimen by determining the expression levels of VEGFA and PLGF, and by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

The present disclosure relates to a method for improving the treatment effect of a chemotherapy regimen of patients suffering from cancer, in particular metastatic pancreatic cancer, by adding bevacizumab to a chemotherapy regimen by determining the expression levels of VEGFA, VEGFR2 and PLGF, and by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

For example, as shown in the appended illustrative example, the following equations can be used for assessing the combined expression level of VEGFA and VEGFR2 or VEGFA and PLGF when the treatment effect is overall survival in patients suffering from metastatic pancreatic cancer.

$$\text{Formula 1 : norm(VEGFA)+1.3*norm(VEGFR2). Cut-point= median or 0}$$

Equivalent formula : VEGFA+3.3*VEGFR2. Cut-point= median or 0 and

$$\text{Formula 2 : 0.25*norm(VEGFA)+0.21*norm(PLGF), cut-point=median or 0}$$

Equivalent formula : 0.19*VEGFA+0.67*PLGF, cut-point= median or 4.8

Where we use log2 transformation and

$$x_i \rightarrow norm(x_i) = \frac{\log 2(x_i) - median(\log 2(x))}{mad(\log 2(x))}$$

Accordingly, in the context of the herein described invention, and in accordance with the appended illustrative example, a high combined expression level of VEGFA and VEGFR2 is (Formula 1 $\geq$ -0.1) and a low combined expression of VEGFA and VEGFR2 is (Formula 1 < -0.1), with regard to overall survival. In the context of the herein described invention, and in accordance with the appended illustrative example, a high combined expression level of VEGFA and PLGF is (Formula 2 $\geq$ -0.042) and a low combined expression of VEGFA and PLGF is (Formula 2 < -0.042), with regard to overall survival. The skilled person will, however, understand that the expression levels measured for markers of a marker panel (or a combined expression level), e.g. for VEGFA and VEGFR2 or VEGFA and PLGF, may be mathematically combined and the combined expression level may be correlated to the underlying diagnostic question in more than one way. Accordingly, marker levels may be combined by any appropriate state of the art mathematical method.

As is also shown in the appended illustrative example, the following equations can be used for assessing the combined expression level of VEGFA and VEGFR2 or VEGFA and PLGF when the treatment effect is progression free survival in patients suffering from metastatic pancreatic cancer.

Formula 1: norm(VEGFA)+1.3*norm(VEGFR2). Cut-point= median or 0

Equivalent formula: VEGFA+3.3*VEGFR2. Cut-point= median or 0
and

Formula 2: 0.25*norm(VEGFA)+0.21*norm(PLGF), cut-point=median or 0

Equivalent formula: 0.19*VEGFA+0.67*PLGF, cut-point= median or 4.8

Where we use log2 transformation and

$$x_i \rightarrow norm(x_i) = \frac{\log 2(x_i) - median(\log 2(x))}{mad(\log 2(x))}$$

Accordingly, in the context of the herein described invention, and in accordance with the appended illustrative example, a high combined expression level of VEGFA and VEGFR2 is (Formula 1 $\geq$ -0.1) and a low combined expression of VEGFA and VEGFR2 is (Formula 1 < -0.1), with regard to progression free survival. In the context of the herein described invention, and in accordance with the appended illustrative example, a high combined expression level of VEGFA and PLGF is (Formula 2 $\geq$ -0.042) and a low combined expression of VEGFA and PLGF is (Formula 2 < -0.042), with regard to progression free survival. The skilled person will, however, understand that the expression levels measured for markers of a marker panel (or a combined expression level), e.g. for VEGFA and VEGFR2 or VEGFA and PLGF, may be mathematically combined and the combined expression level may be correlated to the underlying diagnostic question in more than one way. Accordingly, marker levels may be combined by any appropriate state of the art mathematical method.

For example, as shown in the appended illustrative example, the following equation can be used for assessing the combined expression level of VEGFA, VEGFR2 and PLGF when the treatment effect is overall survival or progression free survival in patients suffering from metastatic pancreatic cancer.

Formula 3:  0.0127 * ln (PLGF+1) + 0.144 * ln (VEGFR2+1) + 0.0949 * ln (VEGFA + 1)

Where ln = log basis e

Accordingly, in the context of the herein described invention, and in accordance with the appended illustrative example, a high combined expression level of VEGFA, VEGFR2 and PLGF is (Formula 3 $\geq$ 0.837) and a low combined expression of VEGFA, VEGFR2 and PLGF is (Formula 3 < 0.837), with regard to overall survival. In the context of the herein described invention, and in accordance with the appended illustrative example, a high combined expression level of VEGFA, VEGFR2 and PLGF is (Formula 3 $\geq$ 0.837) and a low combined expression of VEGFA, VEGFR2 and PLGF is (Formula 3 < 0.837), with regard to progression free survival. The skilled person will, however, understand that the expression levels measured for markers of a marker panel (or a combined expression level), e.g. for VEGFA,VEGFR2 and PLGF, may be mathematically combined and the combined expression level may be correlated to the underlying diagnostic question in more than one way. Accordingly, marker levels may be combined by any appropriate state of the art mathematical method.

The expression level of one or more of the markers VEGFA, VEGFR2 and PLGF may be assessed by any method known in the art suitable for determination of specific protein levels in a patient sample and is preferably determined by an immunoassay method, such as ELISA, employing antibodies specific for one or more of VEGFA, VEGFR2 and PLGF. Such methods are well known and routinely implemented in the art and corresponding commercial antibodies and/or kits are readily available. For example, commercially available antibodies/test kits for VEGFA, VEGFR2 and PLGF can be obtained from Bender RELIATech and R&D Systems as clone 3C5 and 26503 , from R&D systems as clone 89115 and 89109 and from Roche Diagnostics GmbH as clone 2D6D5 and 6A11D2, respectively. Preferably, the expression levels of the marker/indicator proteins of the invention are assessed using the reagents and/or protocol recommendations of the antibody or kit manufacturer. The skilled person will also be aware of further means for determining the expression level of one or more of VEGFA, VEGFR2 and PLGF by immunoassay methods. Therefore, the expression level of one or more of the markers/indicators of the invention can be routinely and reproducibly determined by a person skilled in the art without undue burden. However, to ensure accurate and reproducible results, the invention also encompasses the testing of patient samples in a specialized laboratory that can ensure the validation of testing

procedures.

$VEGF_{121}$ and $VEGF_{110}$ protein can be detected using any method known in the art. For example, tissue or cell samples from mammals can be conveniently assayed for, *e.g.,* proteins using Westerns, ELISAs, etc.Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987)).

If reference is made to the detection or level of $VEGF_{121}$ and $VEGF_{110}$ this means that the sum of both molecules is measured, e.g., using an assay that detects both $VEGF_{121}$ and $VEGF_{110}$. Assays that detect both molecules $VEGF_{121}$ and $VEGF_{110}$ include, e.g., assays that have a sensitivity for the corresponding other form, (i.e. for $VEGF_{121}$ if $VEGF_{110}$ is better recognized, or for $VEGF_{110}$ if $VEGF_{121}$ is better recognized, respectively) of at least 25%. In certain embodiments, in the assays have sensitivity to the corresponding other form of at least 50%, 75%, 80%, 85%, 90% or above. In one embodiment both $VEGF_{121}$ and $VEGF_{110}$ are measured with essentially the same sensitivity.

As to detection of $VEGF_{121}$ and $VEGF_{110}$ protein, various assays are available. For example, the sample may be contacted with an antibody or an antibody combination (e.g. in a sandwich assay) preferentially or specifically binding the short VEGF-A isoforms, $VEGF_{121}$ and $VEGF_{110}$, respectively as compared to the longer naturally occurring VEGF-A isoforms $VEGF_{165}$ and $VEGF_{189}$, respectively. Preferably the short isoforms are detected with an at least 3-fold higher sensitivity as compared to the longer isoforms. An at least 3-fold higher sensitivity is acknowledged if a standard curve is established using a short isoform (purity at least 90% by SDS-PAGE and concentration determined by OD 280nm) and for a long isoform at a predetermined concentration (purity at least 90% by SDS-PAGE and concentration determined by OD 280nm) using the same reagents and the same standard curve the value read of the standard curves is only one third or less of the expected concentration. Also preferred the sensitivity for the short isoforms is at least 4-fold, 5-fold, 6-fold, 7-fold, 8-fold or 9-fold higher as compared to the long isoforms, especially as compared to $VEGF_{165}$.

In one embodiment both short isoforms $VEGF_{121}$ and $VEGF_{110}$ are specifically detected. Such specific detection is e.g. possible if antibodies, especially monoclonal antibodies are used and employed that bind to the sequence generated by joining exons 4 and 8 in $VEGF_{121}$ or the free C-terminal end of $VEGF_{110}$, respectively. Such $VEGF_{110}$ anti C-terminus antibody does not bind to any VEGF-A isoform comprising amino acid 110 as part of a longer polypeptide chain or to shorter VEGF-A fragments ending e.g. at amino acid 109. The monoclonal antibody that binds to the sequence generated by joining exons 4 and 8, respectively, in $VEGF_{121}$ will not bind to the amino acid sequences comprised in the longer VEGF isoforms 165 and 189, respectively, since therein other amino acid sequences are present due to the joining of exon 4 and exon 7, and of exon 4 and exon 5, respectively (see: Ferrara, N., Mol. Biol. of the Cell 21 (2010) 687-690). Specific binding in the above sense is acknowledged, if the antibody used exhibits less than 10% cross-reactivity with a shorter fragment and less than 10% cross-reactivity with those VEGF-A isoforms not having a free C-terminal amino 110 in case of the anti-$VEGF_{110}$ antibody, or those isoforms not comprising the sequence generated by joining exons 4 and 8 in case of the anti-$VEGF_{121}$ antibody, respectively. Also preferred the cross-reactivity will be less than 5%, 4%, 3%, 2% and 1%, respectively, for both shorter fragments and not having a free C-terminal amino acid 110 or VEGF isoforms not having the sequence generated by joining exons 4 and 8, respectively.

Appropriate specific antibodies only binding the short VEGF isoforms $VEGF_{121}$ or $VEGF_{110}$, respectively, can be obtained according to standard procedures. Usually a peptide representing or comprising the C-terminal most at least 4, 5, 6, 7, 8, 9, 10 or more amino acids of $VEGF_{110}$ or a peptide representing or comprising at least 5, 6, 7, 8, 9, 10 or more amino acids comprising amino acids C-terminal and N-terminal to amino acid 115 of $VEGF_{121,}$ respectively, will be synthesized, optionally coupled to a carrier and used for immunization. Specific polyclonal antibodies can be obtained by appropriate immunosorption steps. Monoclonal antibodies can easily be screened for reactivity with $VEGF_{121}$ or $VEGF_{110}$, respectively, and appropriate low cross-reactivity. Low cross-reactivity in terms of the VEGF110-specific antibody can be assessed for both shorter fragments of $VEGF_{110}$ (e.g. lacking the C-terminal amino acid of $VEGF_{110}$) and VEGF-A isoforms not having a free C-terminal amino acid of $VEGF_{110}$. Low cross-reactivity in terms of the $VEGF_{121}$-specific antibody can be assessed using VEGF-isoforms containing the amino acid sequences formed upon joining of exon 4 and exon 7, and of exon 4 and exon 5, respectively.

$VEGF_{111}$ protein or nucleic acids can be detected using any method known in the art. For example, tissue or cell samples from mammals can be conveniently assayed for, *e.g.,* proteins using Westerns, ELISAs, mRNAs or DNAs from a genetic biomarker of interest using Northern, dot-blot, or polymerase chain reaction (PCR) analysis, array hybridization, RNase protection assay, or using DNA SNP chip microarrays, which are commercially available, including DNA microarray snapshots. For example, real-time PCR (RT-PCR) assays such as quantitative PCR assays are well known in the art. In an illustrative embodiment of the disclosure, a method for detecting mRNA from a genetic biomarker of interest in a biological sample comprises producing cDNA from the sample by reverse transcription using at least one primer; amplifying the cDNA so produced; and detecting the presence of the amplified cDNA. In addition, such methods can include one or more steps that allow one to determine the levels of mRNA in a biological sample (e.g., by simultaneously examining the levels a comparative control mRNA sequence of a "housekeeping" gene such as an actin family member). Optionally, the sequence of the amplified cDNA can be determined.

Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987).

As to detection of $VEGF_{111}$ protein, various assays are available For example, the sample may be contacted with an antibody or an antibody combination (e.g. in a sandwich assay) preferentially or specifically binding to $VEGF_{111}$ as compared to the longer naturally occurring VEGF-A isoforms $VEGF_{165}$ and $VEGF_{189}$, respectively. Preferably the short isoform $VEGF_{111}$ is detected with an at least 3-fold higher sensitivity as compared to the longer isoforms. An at least 3-fold higher sensitivity is acknowledged if a standard curve is established using a short isoform (purity at least 90% by SDS-PAGE and concentration determined by OD 280nm) and for a long isoform at a predetermined concentration (purity at least 90% by SDS-PAGE and concentration determined by OD 280nm) using the same reagents and the same standard curve the value read of the standard curves is only one third or less of the expected concentration. Also preferred the sensitivity for the short isoforms is at least 4-fold, 5-fold, 6-fold, 7-fold, 8-fold or 9-fold higher as compared to the long isoforms.

In one embodiment isoform $VEGF_{111}$ is specifically detected. Such specific detection is e.g. possible if antibodies, especially monoclonal antibodies are used and employed that bind to the exon junction unique for $VEGF_{111}$. Such antibody does not bind to other VEGF-A isoform or cleavage products thereof not comprising this specific exon junction. Specific binding in the above sense is acknowledged, if the antibody used exhibits less than 10% cross-reactivity with other VEGF-A isoforms, like $VEGF_{121}$ or $VEGF_{165}$, respectively, not having this unique exon junction. Also preferred the cross-reactivity to e.g. $VEGF_{121}$ will be less than 5%, 4%, 3%, 2% and 1%, respectively.

Specificity for $VEGF_{111}$ in one embodiment is assessed by comparing VEGF111 (purity at least 90% by SDS-PAGE and concentration determined by OD 280nm) and VEGF121 (purity at least 90% by SDS-PAGE and concentration determined by OD 280nm) using the same reagents. If in this comparison the signal obtained for $VEGF_{121}$ material is only one tens or less of the signal as obtained with the $VEGF_{111}$ material, then cross-reactivity towards $VEGF_{121}$ is less than 10%. As the skilled artisan will appreciate the $VEGF_{121}$ signal is preferably read of at a concentration which yields about 50% of the maximal signal for $VEGF_{111}$.

Appropriate specific antibodies only binding the short VEGF isoform $VEGF_{111}$ can be obtained according to standard procedures. Usually a peptide representing or comprising amino acids C-terminal and N-terminal to amino acid 105 of $VEGF_{111}$ will be synthesized, optionally coupled to a carrier and used for immunization. Preferably such peptide will be at least six amino acids long and comprise at least the amino acids 105 and106 of $VEGF_{111}$. Also preferred it will comprise at least the amino acids 104, 105, 106 and 107 of $VEGF_{111}$. As the skilled artisan will appreciate longer peptides comprising e.g. 3 or more amino acids N- and C-terminal to the exon junction between amino acids 105 and 106 of $VEGF_{111}$ can also be used to obtain antibodies specifically binding $VEGF_{111}$.

Unmodified VEGF protein can be detected using any appropriate method known in the art. Preferably an antibody will be used having at least the preferential binding properties to unmodified VEGF as compared to modified VEGF as MAB 3C5, which is commercially available from RELIATech GmbH, Wolfenbüttel, Germany. For example, tissue or cell samples from mammals can be conveniently assayed for the unmodified VEGF protein using Westerns, ELISAs, etc. Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987)).

If reference is made to the detection or level of unmodified VEGF this means that unmodified VEGF-molecules (isoforms or cleavage products) as e.g. bound by MAB 3C5 are measured.

As to detection of unmodified VEGF protein, various assays are available. For example, the sample may be contacted with an antibody or an antibody combination (e.g. in a sandwich assay) preferentially or specifically binding to unmodified VEGF as compared to modified VEGF, e.g. as naturally occurring in a patient's sample. Preferably unmodified VEGF is detected using an antibody specifically binding to unmodified VEGF, i.e., with an antibody having at least 3-fold higher sensitivity for unmodified VEGF165 as compared to modified VEGF165. Such at least 3-fold higher sensitivity for unmodified VEGF is assessed by comparing VEGF165 recombinantly produced in E. coli (purity at least 90% by SDS-PAGE and concentration determined by OD 280nm) and VEGF165 recombinantly produced in HEK cells (purity at least 90% by SDS-PAGE and concentration determined by OD 280nm) using the same reagents. If in this comparison the signal obtained for the HEK-produced material is only one third or less of the signal as obtained with the E. coli-derived material, then unmodified VEGF is detected with an at least 3-fold higher sensitivity. As the skilled artisan will appreciate the signal is preferably read of at about 50% of the maximal signal. Preferably in this assessment the assay of example 5 is used. Also preferred the antibody specifically binding to unmodified VEGF (VEGF165 ex E. coli) is an antibody that detects unmodified VEGF with and at least 4-fold, 5-fold, 6-fold, 7-fold, 8-fold, 9-fold or 10-fold higher sensitivity as compared to the modified VEGF material (VEGF165 ex HEK cells).

In one embodiment unmodified VEGF is specifically detected using an antibody having at least the same binding preference for unmodified VEGF as compared to modified VEGF as the commercially available MAB 3C5. In one embodiment the relative sensitivity for or preferential binding of an antibody to unmodified VEGF is assessed in a sandwich immuno assay, wherein the antibody to unmodified VEGF is used as a capture antibody and a detection antibody is used that binds to an epitope present on all major VEGF-isoforms or cleavage products. In one embodiment the detection antibody will bind to an epitope outside the epitope for MAB 3C5, i.e., it will not bind to an epitope comprised in a synthetic peptide spanning amino acids 33 to 43 of VEGF. Preferably the detection antibody will bind to an epitope comprised in the amino acids ranging from 1 to 32, form 44 to 105, to the last six amino acids of mature VEGF165, or to a conformational epitope not overlapping with the epitope bound by MAB 3C5. In one embodiment the antibody specifically binding unmodified VEGF165 as compared to modified VEGF has the property to bind to an epitope comprised in a synthetic peptide spanning amino acids 33 to 43 of VEGF.

Appropriate specific antibodies specifically binding unmodified VEGF can be obtained according to standard procedures. Usually an isoform of VEGF produced recombinantly in E. coli or obtained synthetically e.g. by solid phase polypeptide synthesis, or a peptide representing or comprising an epitope of VEGF produced recombinantly in E. coli or obtained synthetically e.g. by solid phase polypeptide synthesis will be used as an immunogen. Monoclonal antibodies can easily be produced according to standard protocols and screened for reactivity with unmodified VEGF and appropriate low cross-reactivity with modified VEGF. One convenient and preferred screening method is based on the use of VEGF165 recombinantly produced in E. coli (purity at least 90% by SDS-PAGE and concentration determined by OD 280nm) and of VEGF165 recombinantly produced in HEKcells (purity at least 90% by SDS-PAGE and concentration determined by OD 280nm), respectively.

The expression level of one or more of VEGFA, VEGFR2 and PLGF may be assessed in a patient sample that is a biological sample. The patient sample may be a blood sample, blood serum sample or a blood plasma sample. In one embodiment, the sample is EDTA-plasma. In one embodiment, the sample is citrate-plasma. Methods of obtaining blood samples, blood serum samples and blood plasma samples are well known in the art. The patient sample may be obtained from the patient prior to or after neoadjuvant therapy or prior to or after adjuvant therapy.

In the context of the present disclosure, bevacizumab is to be administered in addition to or as a co-therapy or co-treatment with one or more chemotherapeutic agents administered as part of standard chemotherapy regimen as known in the art. Examples of agents included in such standard chemotherapy regimens include 5-fluorouracil, leucovorin, irinotecan, gemcitabine, erlotinib, capecitabine, taxanes, such as docetaxel and paclitaxel, interferon alpha, vinorelbine, and platinum-based chemotherapeutic agents, such as, carboplatin, cisplatin and oxaliplatin. As demonstrated in the appended illustrative example, the addition of bevacizumab to gemcitabine-erlotinib therapy effected an increase in the overall survival and/or progression free survival in the patients and/or patient population defined and selected according to the expression level of one or more of VEGFA, VEGFR2 and PLGF. Thus, bevacizumab may be combined with a chemotherapy regimen, such as gemcitabine-erlotinib therapy as demonstrated in the appended illustrative example.

Common modes of administration include parenteral administration as a bolus dose or as an infusion over a set period of time, e.g., administration of the total daily dose over 10 min., 20 min., 30 min., 40 min., 50 min., 60 min., 75 min., 90 min., 105 min., 120 min., 3 hr., 4 hr., 5 hr. or 6 hr. For example, 2.5 mg/kg of body weight to 15 mg/kg of body weight bevacizumab (Avastin®) can be administered every week, every 2 weeks or every 3 weeks, depending on the type of cancer being treated. Examples of dosages include 2.5 mg/kg of body weight, 5 mg/kg of body weight, 7.5 mg/kg of body weight, 10 mg/kg of body weight and 15 mg/kg of body weight given every week, every 2 weeks or every 3 weeks. Further examples of dosages are 5 mg/kg of body weight every 2 weeks, 10 mg/kg every 2 weeks of body weight, 7.5 mg/kg of body weight every 3 weeks and 15 mg/kg of body weight every 3 weeks. For the treatment of pancreatic cancer, in particular metastatic pancreatic cancer, dosages include 5 mg/kg of body weight every 2 weeks, 10 mg/kg every 2 weeks, 7.5 mg/kg of body weight every 3 weeks and 15 mg/kg of body weight every 3 weeks. The skilled person will recognize that further modes of administration of bevacizumab are encompassed by the disclosure as determined by the specific patient and chemotherapy regimen, and that the specific mode of administration and therapeutic dosage are best determined by the treating physician according to methods known in the art.

The patients selected according to the methods of the present disclosure are treated with bevacizumab in combination with a chemotherapy regimen, and may be further treated with one or more additional anti-cancer therapies. In certain aspects, the one or more additional anti-cancer therapy is radiation.

The present disclosure also relates to a diagnostic composition or kit comprising oligonucleotides or polypeptides suitable for the determination of expression levels of one or more of VEGFA, VEGFR2 and PLGF. As detailed herein, oligonucleotides such as DNA, RNA or mixtures of DNA and RNA probes may be of use in detecting mRNA levels of the marker/indicator proteins, while polypeptides may be of use in directly detecting protein levels of the marker/indicator proteins via specific protein-protein interaction. In preferred aspects of the disclosure, the polypeptides encompassed as probes for the expression levels of one or more of VEGFA, VEGFR2 and PLGF, and included in the kits or diagnostic compositions described herein, are antibodies specific for these proteins, or specific for homologues and/or truncations thereof.

Accordingly, in a further embodiment of the present disclosure provides a kit useful for carrying out the methods herein described, comprising oligonucleotides or polypeptides capable of determining the expression level of one or more of VEGFA, VEGFR2 and PLGF. The oligonucleotides may comprise primers and/or probes specific for the mRNA encoding one or more of the markers/indicators described herein, and the polypeptides comprise proteins capable of specific interaction with the marker/indicator proteins, e.g., marker/indicator specific antibodies or antibody fragments.

Accordingly, the present disclosure relates to bevacizumab for use in an improved chemotherapy regimen for a patient suffering from pancreatic cancer wherein the expression level of one or more of VEGFA, VEGFR2 and PLGF in a patient sample in determined whereby a patient having an increased level of one or more of VEGFA VEGFR2 and PLGF relative to control levels determined in patients diagnosed with pancreatic cancer is administered bevacizumab in addition to the chemotherapy regimen.

The following similar uses can be applied mutatis mutandis.

The present disclosure relates to the use of bevacizumab for improving the treatment effect of a chemotherapy regimen of a patient suffering from pancreatic cancer comprising the following steps:

(a) determining the expression level of one or more of VEGFA, VEGFR2 and PLGF in a patient sample; and
(b) administering bevacizumab in combination with the chemotherapy regimen to the patient having an increased level of one or more of VEGFA VEGFR2 and PLGF relative to control levels determined in patients diagnosed with pancreatic cancer.

The pancreatic cancer may be metastatic pancreatic cancer. The chemotherapy regimen may be gemcitabine-erlotinib therapy.

Accordingly, the present disclosure relates to the use of bevacizumab for improving the treatment effect of a chemotherapy regimen of a patient suffering from pancreatic cancer comprising the following steps:

(a) obtaining a sample from said patient;
(b) determining the expression level of one or more of VEGFA, VEGFR2 and PLGF; and
(c) administering bevacizumab in combination with the chemotherapy regimen to the patient having an increased level of one or more of VEGFA, VEGFR2 and PLGF relative to control levels determined in patients diagnosed pancreatic cancer.

The pancreatic cancer may be metastatic pancreatic cancer. The chemotherapy regimen may be gemcitabine-erlotinib therapy.

The present disclosure relates to the use of bevacizumab for improving the overall survival of a patient suffering from pancreatic cancer comprising the following steps:

(a) determining the expression level of one or more of VEGFA, VEGFR2 and PLGF in a patient sample; and
(b) administering bevacizumab in combination with a chemotherapy regimen to the patient having an increased level of one or more of VEGFA VEGFR2 and PLGF relative to control levels determined in patients diagnosed with pancreatic cancer.

The pancreatic cancer may be metastatic pancreatic cancer. The chemotherapy regimen may be gemcitabine-erlotinib therapy.

Accordingly, the present disclosure relates to the use of bevacizumab for improving the overall survival of a patient suffering from pancreatic cancer comprising the following steps:

(a) obtaining a sample from said patient;
(b) determining the expression level of one or more of VEGFA, VEGFR2 and PLGF; and
(c) administering bevacizumab in combination with a chemotherapy regimen to the patient having an increased level of one or more of VEGFA, VEGFR2 and PLGF relative to control levels determined in patients diagnosed pancreatic cancer.

The pancreatic cancer may be metastatic pancreatic cancer. The chemotherapy regimen may be gemcitabine-erlotinib therapy.

The present disclosure relates to the use of bevacizumab for improving the progression free survival of a patient suffering from pancreatic cancer comprising the following steps:

(a) determining the expression level of one or more of VEGFA, VEGFR2 and PLGF in a patient sample; and

(b) administering bevacizumab in combination with the chemotherapy regimen to the patient having an increased level of one or more of VEGFA VEGFR2 and PLGF relative to control levels determined in patients diagnosed with pancreatic cancer.

The pancreatic cancer may be metastatic pancreatic cancer. The chemotherapy regimen may be gemcitabine-erlotinib therapy.

Accordingly, the present disclosure relates to the use of bevacizumab for improving the progression free survival of a patient suffering from pancreatic cancer comprising the following steps:

(a) obtaining a sample from said patient;

(b) determining the expression level of one or more of VEGFA, VEGFR2 and PLGF; and

(c) administering bevacizumab in combination with the chemotherapy regimen to the patient having an increased level of one or more of VEGFA, VEGFR2 and PLGF relative to control levels determined in patients diagnosed pancreatic cancer.

The pancreatic cancer may be metastatic pancreatic cancer. The chemotherapy regimen may be gemcitabine-erlotinib therapy.

The present disclosure relates to the use of bevacizumab for improving the overall survival of a patient suffering from pancreatic cancer comprising the following steps:

(a) determining the expression level of VEGFA or VEGFR2 in a patient sample; and

(b) administering bevacizumab in combination with a chemotherapy regimen to the patient having an increased level of VEGFA or VEGFR2 relative to control levels determined in patients diagnosed with pancreatic cancer.

The pancreatic cancer may be metastatic pancreatic cancer. The chemotherapy regimen may be gemcitabine-erlotinib therapy.

Accordingly, the present disclosure relates to the use of bevacizumab for improving the overall survival of a patient suffering from pancreatic cancer comprising the following steps:

(a) obtaining a sample from said patient;

(b) determining the expression level of VEGFA or VEGFR2; and

(c) administering bevacizumab in combination with a chemotherapy regimen to the patient having an increased level of VEGFA or VEGFR2 relative to control levels determined in patients diagnosed pancreatic cancer.

The pancreatic cancer may be metastatic pancreatic cancer. The chemotherapy regimen may be gemcitabine-erlotinib therapy.

The present disclosure relates to the use of bevacizumab for improving the progression free survival of a patient suffering from pancreatic cancer comprising the following steps:

(a) determining the expression level of VEGFA or PLGF in a patient sample; and

(b) administering bevacizumab in combination with the chemotherapy regimen to the patient having an increased level of VEGFA or PLGF relative to control levels determined in patients diagnosed with pancreatic cancer.

The pancreatic cancer may be metastatic pancreatic cancer. The chemotherapy regimen may be gemcitabine-erlotinib therapy.

Accordingly, the present disclosure relates to the use of bevacizumab for improving the progression free survival of a patient suffering from pancreatic cancer comprising the following steps:

(a) obtaining a sample from said patient;

(b) determining the expression level of VEGFA or PLGF; and

(c) administering bevacizumab in combination with the chemotherapy regimen to the patient having an increased level of VEGFA or PLGF relative to control levels determined in patients diagnosed pancreatic cancer.

The pancreatic cancer may be metastatic pancreatic cancer. The chemotherapy regimen may be gemcitabine-erlotinib therapy.

The present disclosure provides the use of bevacizumab for improving overall survival of a patient suffering from metastatic pancreatic cancer comprising the following steps:

(a) determining the expression level of VEGFA and VEGFR2 in a patient sample; and

(b) administering bevacizumab in combination with a chemotherapy regimen to the patient having an increased combined expression level of VEGFA and VEGFR2 relative to a control combined expression level determined in patients diagnosed with metastatic pancreatic cancer.

The present disclosure relates to the use of bevacizumab for improving overall survival of a patient suffering from metastatic pancreatic cancer comprising the following steps:

(a) obtaining a sample from said patient;

(b) determining the expression level of VEGFA and VEGFR2; and

(c) administering bevacizumab in combination with a chemotherapy regimen to the patient having an increased combined expression level of VEGFA and VEGFR2 relative to a control combined expression level determined in patients diagnosed with metastatic pancreatic cancer.

The disclosure relates to the use of bevacizumab for improving overall survival of a patient suffering from metastatic pancreatic cancer comprising the following steps:

(a) determining the expression level of VEGFA and VEGFR2 in a patient sample; and

(b) administering bevacizumab in combination with a chemotherapy regimen to the patient having an increased combined expression level of VEGFA and VEGFR2 relative to a control combined expression level determined in patients diagnosed with metastatic pancreatic cancer,

wherein the chemotherapy regimen is gemcitabine-erlotinib therapy.

Accordingly, the present disclosure relates to the use of bevacizumab for overall survival of a patient suffering from metastatic pancreatic cancer comprising the following steps:

(a) obtaining a sample from said patient;

(b) determining the expression level of VEGFA and VEGFR2; and

(c) administering bevacizumab in combination with a chemotherapy regimen to the patient having an increased combined expression level of VEGFA and VEGFR2 relative to a control combined expression level determined in patients diagnosed with metastatic pancreatic cancer,

wherein the chemotherapy regimen is gemcitabine-erlotinib therapy.

The present disclosure provides the use of bevacizumab for improving progression free survival of a patient suffering from metastatic pancreatic cancer comprising the following steps:

(a) determining the expression level of VEGFA and VEGFR2 in a patient sample; and

(b) administering bevacizumab in combination with a chemotherapy regimen to the patient having an increased combined expression level of VEGFA and VEGFR2 relative to a control combined expression level determined in patients diagnosed with metastatic pancreatic cancer.

The present disclosure relates to the use of bevacizumab for improving progression free survival of a patient suffering from metastatic pancreatic cancer comprising the following steps:

(a) obtaining a sample from said patient;

(b) determining the expression level of VEGFA and VEGFR2; and

(c) administering bevacizumab in combination with a chemotherapy regimen to the patient having an increased combined expression level of VEGFA and VEGFR2 relative to a control combined expression level determined in patients diagnosed with metastatic pancreatic cancer.

The disclosure relates to the use of bevacizumab for improving progression free survival of a patient suffering from metastatic pancreatic cancer comprising the following steps:

(a) determining the expression level of VEGFA and VEGFR2 in a patient sample; and

(b) administering bevacizumab in combination with a chemotherapy regimen to the patient having an increased combined expression level of VEGFA and VEGFR2 relative to a control combined expression level determined in patients diagnosed with metastatic pancreatic cancer,

wherein the chemotherapy regimen is gemcitabine-erlotinib therapy.

Accordingly, the present disclosure relates to the use of bevacizumab for progression free survival of a patient suffering from metastatic pancreatic cancer comprising the following steps:

(a) obtaining a sample from said patient;
(b) determining the expression level of VEGFA and VEGFR2; and
(c) administering bevacizumab in combination with a chemotherapy regimen to the patient having an increased combined expression level of VEGFA and VEGFR2 relative to a control combined expression level determined in patients diagnosed with metastatic pancreatic cancer,
wherein the chemotherapy regimen is gemcitabine-erlotinib therapy.

The present disclosure provides the use of bevacizumab for improving overall survival of a patient suffering from metastatic pancreatic cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with metastatic pancreatic cancer.

Accordingly, the present disclosure relates to the use of bevacizumab for overall survival of a patient suffering from metastatic pancreatic cancer comprising the following steps:

(a) obtaining a sample from said patient;
(b) determining the expression level of VEGFA and PLGF; and
(c) administering bevacizumab in combination with a chemotherapy regimen to the patient having an increased combined expression level of VEGFA and PLGF relative to a control combined expression level determined in patients diagnosed with metastatic pancreatic cancer.

The present disclosure provides the use of bevacizumab for improving overall survival of a patient suffering from metastatic pancreatic cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with metastatic pancreatic cancer,

wherein the chemotherapy regimen is gemcitabine-erlotinib therapy.
Accordingly, the present disclosure relates to the use of bevacizumab for overall survival of a patient suffering from metastatic pancreatic cancer comprising the following steps:

(a) obtaining a sample from said patient;
(b) determining the expression level of VEGFA and PLGF; and
(c) administering bevacizumab in combination with a chemotherapy regimen to the patient having an increased combined expression level of VEGFA and PLGF relative to a control combined expression level determined in patients diagnosed with metastatic pancreatic cancer
wherein the chemotherapy regimen is gemcitabine-erlotinib therapy.

The present disclosure provides the use of bevacizumab for improving progression free survival of a patient suffering from metastatic pancreatic cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with metastatic pancreatic cancer.

Accordingly, the present disclosure relates to the use of bevacizumab for progression free survival of a patient suffering from metastatic pancreatic cancer comprising the following steps:

(a) obtaining a sample from said patient;

(b) determining the expression level of VEGFA and PLGF; and

(c) administering bevacizumab in combination with a chemotherapy regimen to the patient having an increased combined expression level of VEGFA and PLGF relative to a control combined expression level determined in patients diagnosed with metastatic pancreatic cancer.

The present disclosure provides the use of bevacizumab for improving progression free survival of a patient suffering from metastatic pancreatic cancer comprising the following steps:

(a) determining the expression level of VEGFA and PLGF in a patient sample; and

(b) administering bevacizumab in combination with a chemotherapy regimen to the patient having an increased combined expression level of VEGFA and PLGF relative to a control combined expression level determined in patients diagnosed with metastatic pancreatic cancer, wherein the chemotherapy regimen is gemcitabine-erlotinib therapy.

Accordingly, the present disclosure relates to the use of bevacizumab for progression free survival of a patient suffering from metastatic pancreatic cancer comprising the following steps:

(a) obtaining a sample from said patient;

(b) determining the expression level of VEGFA and PLGF; and

(c) administering bevacizumab in combination with a chemotherapy regimen to the patient having an increased combined expression level of VEGFA and PLGF relative to a control combined expression level determined in patients diagnosed with metastatic pancreatic cancer wherein the chemotherapy regimen is gemcitabine-erlotinib therapy.

The present disclosure provides the use of bevacizumab for improving overall survival of a patient suffering from metastatic pancreatic cancer comprising the following steps:

(a) determining the expression level of VEGFA, VEGFR2 and PLGF in a patient sample; and

(b) administering bevacizumab in combination with a chemotherapy regimen to the patient having an increased combined expression level of VEGFA, VEGFR2 and PLGF relative to a control combined expression level determined in patients diagnosed with metastatic pancreatic cancer.

The present disclosure relates to the use of bevacizumab for improving overall survival of a patient suffering from metastatic pancreatic cancer comprising the following steps:

(a) obtaining a sample from said patient;

(b) determining the expression level of VEGFA, VEGFR2 and PLGF; and

(c) administering bevacizumab in combination with a chemotherapy regimen to the patient having an increased combined expression level of VEGFA, VEGFR2 and PLGF relative to a control combined expression level determined in patients diagnosed with metastatic pancreatic cancer.

The disclosure relates to the use of bevacizumab for improving overall survival of a patient suffering from metastatic pancreatic cancer comprising the following steps:

(a) determining the expression level of VEGFA, VEGFR2 and PLGF in a patient sample; and

(b) administering bevacizumab in combination with a chemotherapy regimen to the patient having an increased combined expression level of VEGFA, VEGFR2 and PLGF relative to a control combined expression level determined in patients diagnosed with metastatic pancreatic cancer, wherein the chemotherapy regimen is gemcitabine-erlotinib therapy.

Accordingly, the present disclosure relates to the use of bevacizumab for overall survival of a patient suffering from metastatic pancreatic cancer comprising the following steps:

(a) obtaining a sample from said patient;

(b) determining the expression level of VEGFA, VEGFR2 and PLGF; and

(c) administering bevacizumab in combination with a chemotherapy regimen to the patient having an increased combined expression level of VEGFA, VEGFR2 and PLGF relative to a control combined expression level determined in patients diagnosed with metastatic pancreatic cancer,

wherein the chemotherapy regimen is gemcitabine-erlotinib therapy.

The present disclosure provides the use of bevacizumab for improving progression free survival of a patient suffering from metastatic pancreatic cancer comprising the following steps:

(a) determining the expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with metastatic pancreatic cancer.

The present disclosure relates to the use of bevacizumab for improving progression free survival of a patient suffering from metastatic pancreatic cancer comprising the following steps:

(a) obtaining a sample from said patient;
(b) determining the expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with metastatic pancreatic cancer.

The disclosure relates to the use of bevacizumab for improving progression free survival of a patient suffering from metastatic pancreatic cancer comprising the following steps:

(a) determining the expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with metastatic pancreatic cancer,
wherein the chemotherapy regimen is gemcitabine-erlotinib therapy.

Accordingly, the present disclosure relates to the use of bevacizumab for progression free survival of a patient suffering from metastatic pancreatic cancer comprising the following steps:

(a) obtaining a sample from said patient;
(b) determining the expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with metastatic pancreatic cancer,
wherein the chemotherapy regimen is gemcitabine-erlotinib therapy.

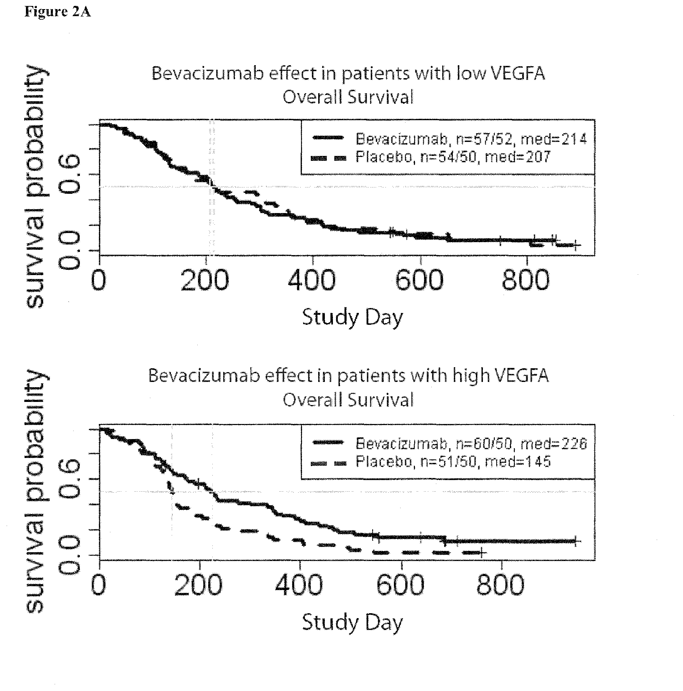

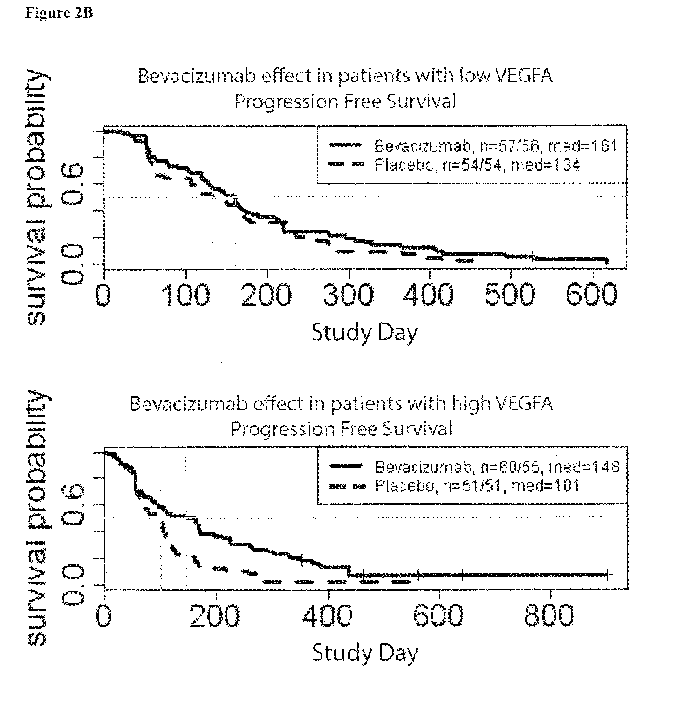

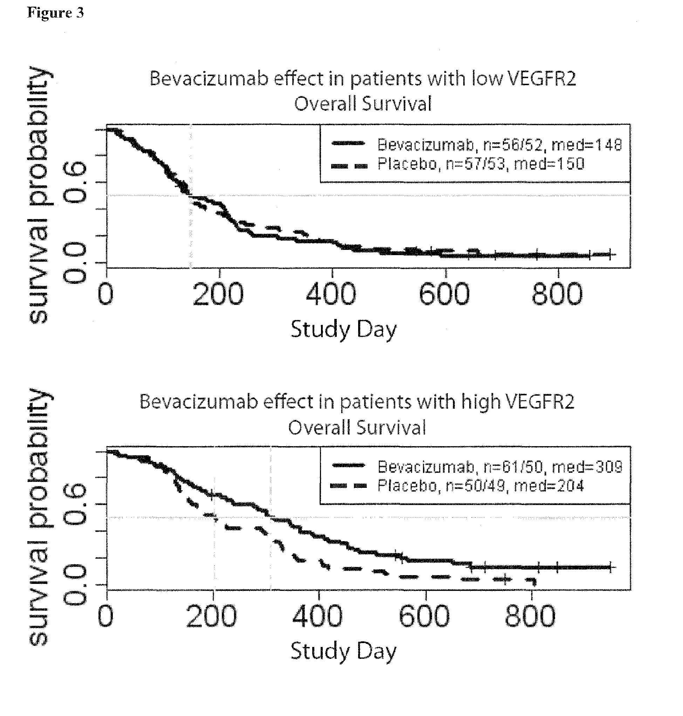

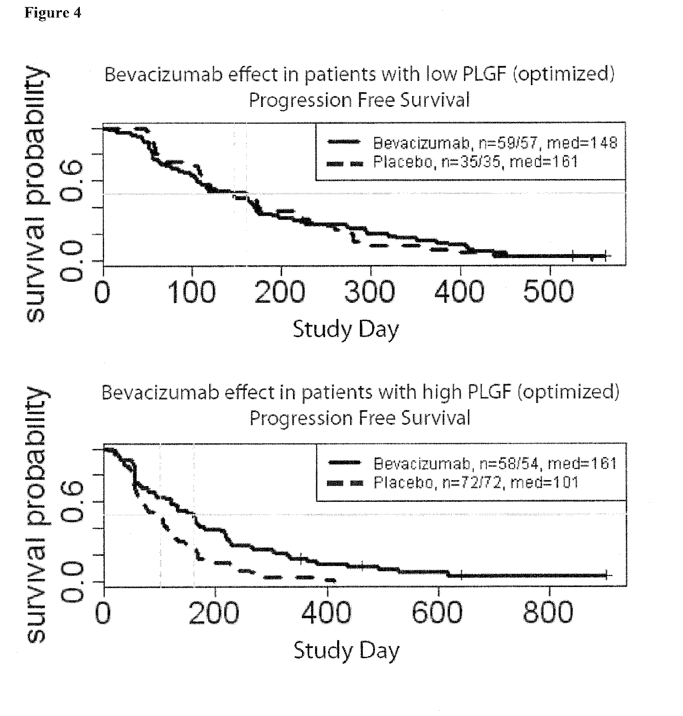

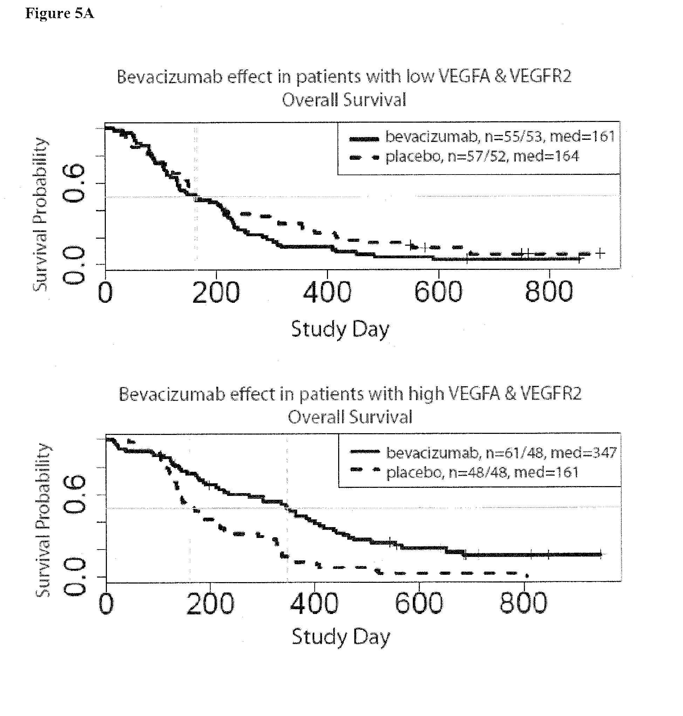

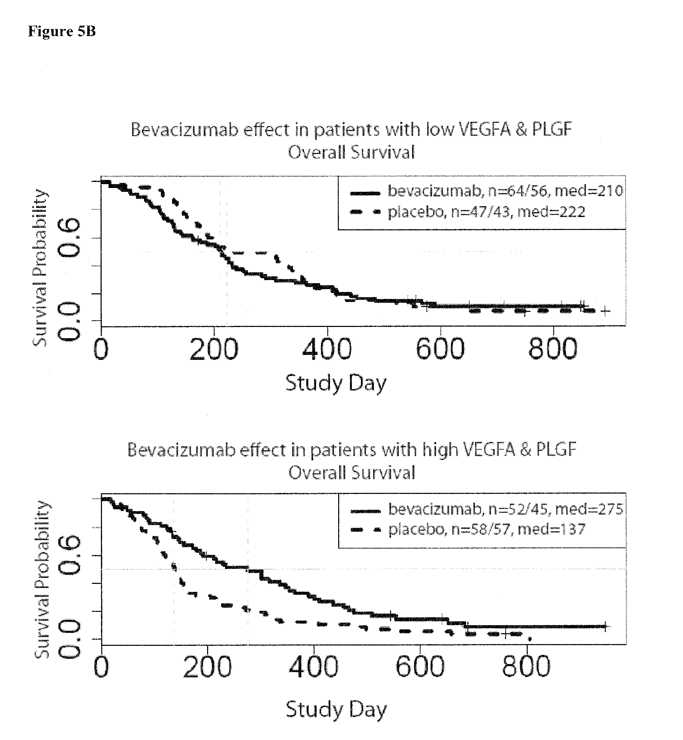

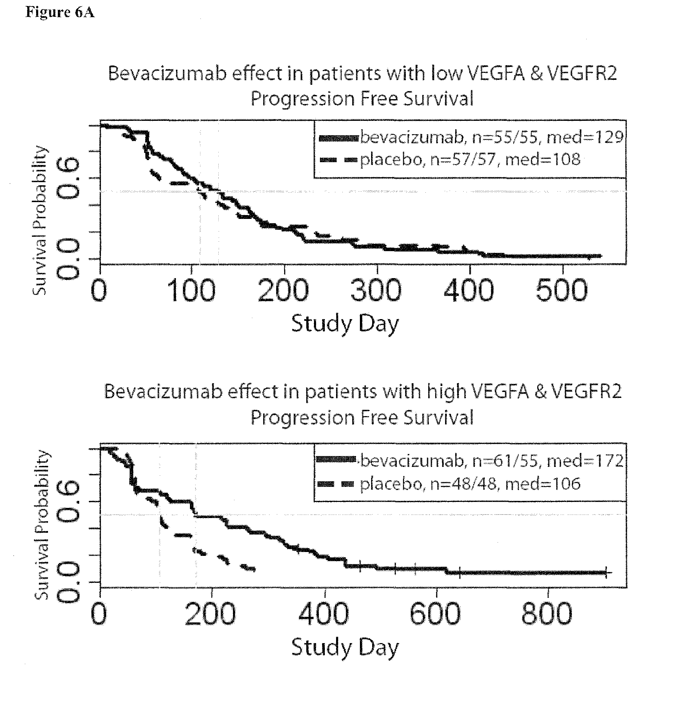

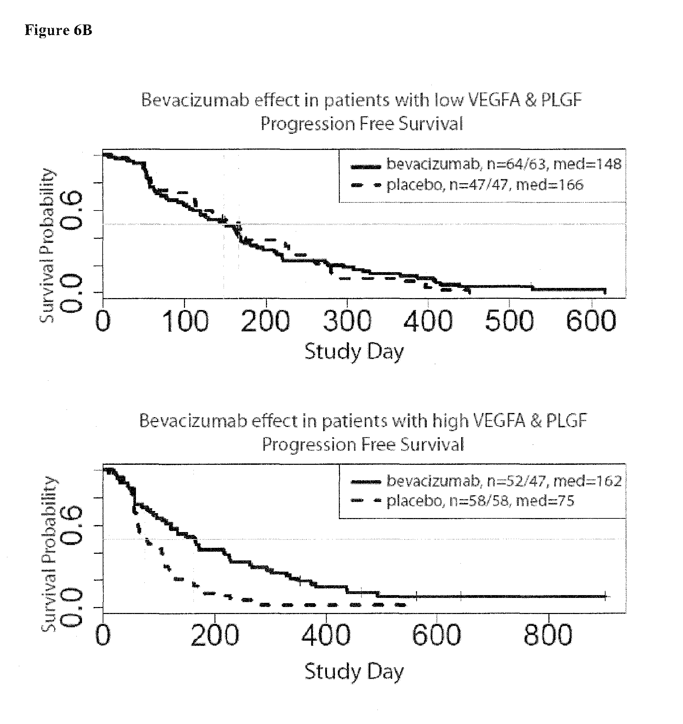

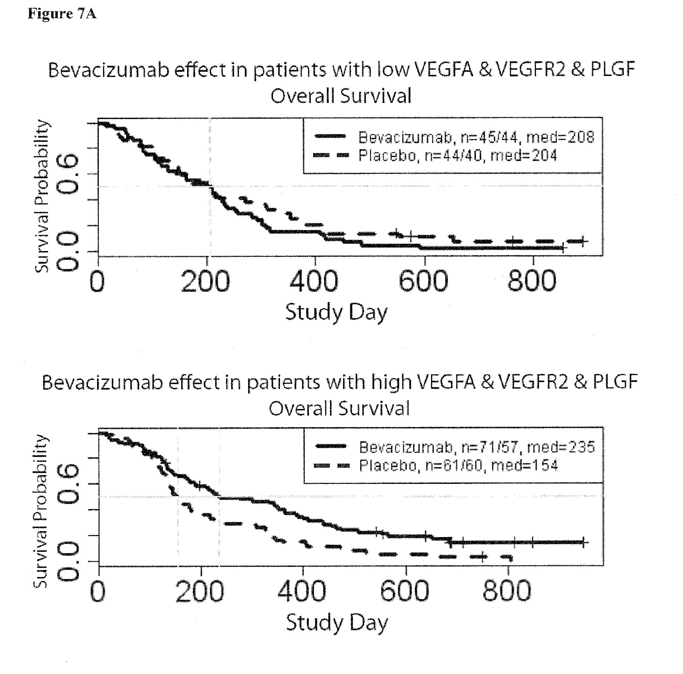

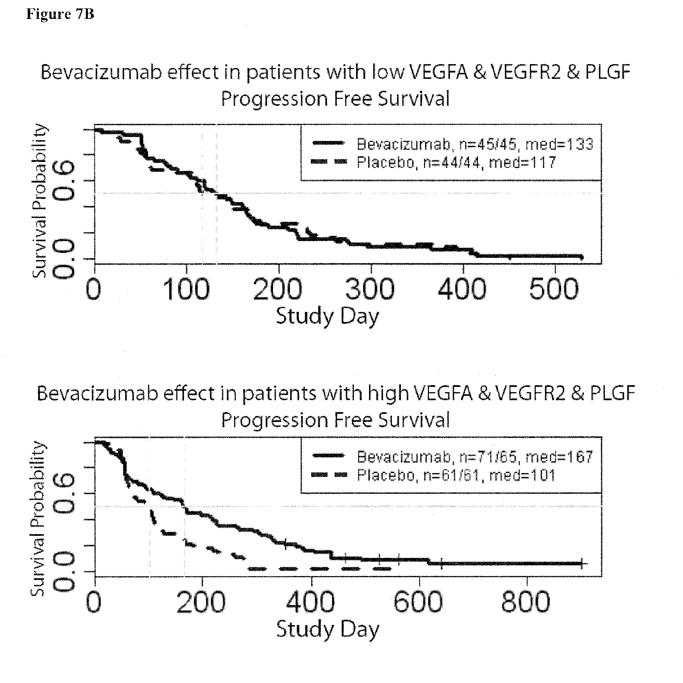

As documented in the appended illustrative example, the present disclosure solves the identified technical problem in that it could surprisingly be shown that the expression levels of one or more of VEGFA, VEGFR2 and PLGF in a given patient, relative to control levels determined in patients diagnosed with pancreatic cancer, in particular metastatic pancreatic cancer, correlate with treatment effect in patients administered bevacizumab in combination with a gemcitabine-erlotinib chemotherapy regimen. As is shown in the appended illustrative example, it was surprisingly found that an increased protein expression level of VEGFA or VEGFR2 correlated with improved overall survival of patients suffering from metastatic pancreatic cancer that were treated with bevacizumab and a gemcitabine-erlotinib chemotherapy regimen in comparison to patients treated with placebo and a gemcitabine-erlotinib chemotherapy regimen (Figures 2 and 3). It was surprisingly found that an increased protein expression level of VEGFA or PLGF correlated with improved progression free survival of patients suffering from metastatic pancreatic cancer that were treated with bevacizumab and a gemcitabine-erlotinib chemotherapy regimen in comparison to patients treated with placebo and a gemcitabine-erlotinib chemotherapy (Figures 2 and 4). It was further surprisingly found that an increased combined expression level of VEGFA and VEGFR2 correlated with improved overall survival and progression free survival of patients suffering from metastatic pancreatic cancer that were treated with bevacizumab and a gemcitabine-erlotinib chemotherapy regimen in comparison to patients treated with placebo and a gemcitabine-erlotinib chemotherapy regimen (Figures 5 and 6). It was also surprisingly found that an increased combined expression level of VEGFA and PLGF correlated with improved overall survival and progression free survival in patients suffering from metastatic pancreatic cancer that were treated with bevacizumab and a gemcitabine-erlotinib chemotherapy regimen in comparison to patients treated with placebo and a gemcitabine-erlotinib chemotherapy regimen (Figures 5 and 6). It was further surprisingly found that an increased combined expression level of VEGFA, VEGFR2 and PLGF correlated with improved overall survival and progression free survival in patients suffering from metastatic pancreatic cancer that were treated with bevacizumab and a gemcitabine-erlotinib chemotherapy regimen in comparison to patients treated with placebo and a gemcitabine-erlotinib chemotherapy regimen (Figure 7). These results are particularly surprising in that these individual markers and the above described combinations of these markers showed no correlation with overall survival when analysed in patient blood plasma samples from a study comparing docetaxel therapy plus bevacizumab or placebo in patients suffering from locally advanced, recurrent or metastatic HER-2 negative breast cancer.

The disclosure, therefore, relates to an in vitro method of predicting the response to or sensitivity to the addition of bevacizumab to a chemotherapy regimen of a patient suffering from, suspect to suffer from or prone to suffer from pancreatic cancer, in particular metastatic pancreatic cancer, comprising determining the expression level, including combined expression levels, of one or more of VEGFA, VEGFR2 and PLGF in a patient sample. Accordingly, in the context of the methods described herein, the disclosure provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from pancreatic cancer, in particular metastatic pancreatic cancer, comprising determining the expression level, including combined expression levels, of one or more of VEGFA, VEGFR2 and PLGF in a patient sample.

The disclosure, therefore, provides an in vitro method of predicting the response to or sensitivity to the addition of bevacizumab to a chemotherapy regimen of a patient suffering from, suspected to suffer from, or prone to suffer from metastatic pancreatic cancer comprising determining the combined expression level of VEGFA and VEGFR2 in a patient sample. Accordingly, in the context of the methods described herein, the disclosure provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from metastatic pancreatic cancer comprising determining the combined expression level of VEGFA and VEGFR2 in a patient sample.

The disclosure provides an in vitro method of predicting the response to or sensitivity to the addition of bevacizumab to a chemotherapy regimen of a patient suffering from, suspected to suffer from, or prone to suffer from metastatic pancreatic cancer comprising determining the combined expression level of VEGFA and PLGF in a patient sample. Accordingly, in the context of the methods described herein, the disclosure provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from metastatic pancreatic cancer comprising determining the combined expression level of VEGFA and PLGF in a patient sample.

The disclosure provides an in vitro method of predicting the response to or sensitivity to the addition of bevacizumab to a chemotherapy regimen of a patient suffering from, suspected to suffer from, or prone to suffer from metastatic pancreatic cancer comprising determining the combined expression level of VEGFA, VEGFR2 and PLGF in a patient sample. Accordingly, in the context of the methods described herein, the disclosure provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from metastatic pancreatic cancer comprising determining the combined expression level of VEGFA, VEGFR2 and PLGF in a patient sample.

The phrase "responsive to" in the context of the present invention indicates that a subject/patient suffering, suspected to suffer or prone to suffer from pancreatic cancer, in particular metastatic pancreatic cancer, shows a response to a chemotherapy regimen comprising the addition of bevacizumab. A skilled person will readily be in a position to determine whether a person treated with bevacizumab according to the methods of the invention shows a response. For example, a response may be reflected by decreased suffering from the metastatic pancreatic cancer, such as a diminished and/or halted tumor growth, reduction of the size of a tumor, and/or amelioration of one or more symptoms of the cancer. Preferably, the response may be reflected by decreased or diminished indices of the metastatic conversion of the pancreatic cancer such as the prevention of the formation of metastases or a reduction of number or size of metastases (*see, e.g.,* Eisenhauser et al., New response evaluation criteria in solid tumours: Revised RECIST guideline (version 1.1) Eur. J. Cancer 2009 45: 228-247).

The phrase "sensitive to" in the context of the present invention indicates that a subject/patient suffering, suspected to suffer or prone to suffer from pancreatic cancer, in particular metastatic pancreatic cancer, shows in some way a positive reaction to treatment with bevacizumab in combination with a chemotherapy regimen. The reaction of the patient may be less pronounced when compared to a patient "responsive to" as described hereinabove. For example, the patient may experience less suffering associated with the disease, though no reduction in tumor growth or metastatic indicator may be measured, and/or the reaction of the patient to the bevacizumab in combination with the chemotherapy regimen may be only of a transient nature, i.e., the growth of (a) tumor and/or (a) metastasis(es) may only be temporarily reduced or halted.

The phrase "a patient suffering from" in accordance with the invention refers to a patient showing clinical signs of pancreatic cancer, in particular metastatic pancreatic cancer. The phrases "suspected to suffer from", "being susceptible to", "prone to suffer from" or "being prone to", in the context of metastatic pancreatic cancer, refers to an indication disease in a patient based on, *e.g.,* a possible genetic predisposition, a pre- or eventual exposure to hazardous and/or carcinogenic compounds, or exposure to carcinogenic physical hazards, such as radiation.

The phrase "treatment effect of a chemotherapy regimen" in the context of the present invention encompasses the terms "overall survival" and "progression-free survival".

The phrase "overall survival" in the context of the present invention refers to the length of time during and after treatment the patient survives. As the skilled person will appreciate, a patient's overall survival is improved or enhanced, if the patient belongs to a subgroup of patients that has a statistically significant longer mean survival time as compared to another subgroup of patients.

The phrase "progression-free survival" in the context of the present invention refers to the length of time during and after treatment during which, according to the assessment of the treating physician or investigator, the patient's disease does not become worse, i.e., does not progress. As the skilled person will appreciate, a patient's progression-free survival is improved or enhanced if the patient experiences a longer length of time during which the disease does not progress as compared to the average or mean progression free survival time of a control group of similarly situated patients.

The terms "administration" or "administering" as used herein mean the administration of an angiogenesis inhibitor, e.g., bevacizumab (Avastin®), and/or a pharmaceutical composition/treatment regimen comprising an angiogenesis inhibitor, e.g., bevacizumab (Avastin®), to a patient in need of such treatment or medical intervention by any suitable means known in the art for administration of a therapeutic antibody. Nonlimiting routes of administration include by oral, intravenous, intraperitoneal, subcutaneous, intramuscular, tropical, intradermal, intranasal or intrabronchial administration (for example as effected by inhalation). Particularly preferred in context of this disclosure is parenteral administration, e.g., intravenous administration.

The term "antibody" is herein used in the broadest sense and includes, but is not limited to, monoclonal and polyclonal antibodies, multispecific antibodies (e.g., bispecific antibodies), chimeric antibodies, CDR grafted antibodies, humanized antibodies, camelized antibodies, single chain antibodies and antibody fragments and fragment constructs, *e.g.*, $F(ab')_2$ fragments, Fab-fragments, Fv-fragments, single chain Fv-fragments (scFvs), bispecific scFvs, diabodies, single domain antibodies (dAbs) and minibodies, which exhibit the desired biological activity, in particular, specific binding to one or more of VEGFA, VEGFR2 and PLGF, or to homologues, variants, fragments and/or isoforms thereof.

The term "aptamer" is herein used in the broadest sense and includes, but is not limited to, oligonucleotides, including RNA, DNA and RNA/DNA molecules, or peptide molecules, which exhibit the desired biological activity, in particular, specific binding to one or more of VEGFA, VEGFR2 and PLGF, or to homologues, variants, fragments and/or isoforms thereof.

As used herein "chemotherapy regimen" or "chemotherapeutic agent" include any active agent that can provide an anticancer therapeutic effect and may be a chemical agent or a biological agent, in particular, that are capable of interfering with cancer or tumor cells. Preferred active agents are those that act as anti-neoplastic (chemotoxic or chemostatic) agents which inhibit or prevent the development, maturation or proliferation of malignant cells. Nonlimiting examples of a chemotherapy regimen or chemotherapeutic agents include alkylating agents such as nitrogen mustards (*e.g.*, mechlorethamine, cyclophosphamide, ifosfamide, melphalan and chlorambucil), nitrosoureas (*e.g.*, carmustine (BCNU), lomustine (CCNU), and semustine (methyl-CCNU)), ethylenimines/ methylmelamines (*e.g.*, thriethylenemelamine (TEM), triethylene, thiophosphoramide (thiotepa), hexamethylmelamine (HMM, altretamine)), alkyl sulfonates (*e.g.*, busulfan), and triazines (*e.g., * dacarbazine (DTIC)); antimetabolites such as folic acid analogs (*e.g.,* methotrexate, trimetrexate), pyrimidine analogs (e.g., 5-fluorouracil, capecitabine, fluorodeoxyuridine, gemcitabine, cytosine arabinoside (AraC, cytarabine), 5-azacytidine, 2,2'-difluorodeoxycytidine), and purine analogs (*e.g.*, 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA)); antimitotic drugs developed from natural products *(e.g.,* paclitaxel, vinca alkaloids *(e.g.,* vinblastine (VLB), vincristine, and vinorelbine), docetaxel, estramustine, and estramustine phosphate), epipodophylotoxins (*.e.g,* etoposide, teniposide), antibiotics (*.e.g* actimomycin D, daunomycin (rubidomycin), daunorubicon, doxorubicin, epirubicin, mitoxantrone, idarubicin, bleomycins, plicamycin (mithramycin), mitomycinC, actinomycin), enzymes *(e.g.,* L-asparaginase), and biological response modifiers *(e.g.,* interferon-alpha, IL-2, G-CSF, GM-CSF); miscellaneous agents including platinum coordination complexes (e.g., cisplatin, carboplatin, oxaliplatin), anthracenediones (e.g., mitoxantrone), substituted urea (*i.e.,* hydroxyurea), methylhydrazine derivatives *(e.g.,* N-methylhydrazine (MIH), procarbazine), adrenocortical suppressants (e.g., mitotane (o,p'-DDD, aminoglutethimide); hormones and antagonists including adrenocorticosteroid antagonists (*.e.g* prednisone and equivalents, dexamethasone, aminoglutethimide), progestins (e.g., hydroxyprogesterone caproate, medroxyprogesterone acetate, megestrol acetate), estrogens (*e.g.,* diethylstilbestrol, ethinyl estradiol and equivalents thereof); antiestrogens (e.g., tamoxifen), androgens (e.g., testosterone propionate, fluoxymesterone and equivalents thereof), antiandrogens (e.g., flutamide, gonadotropin-releasing hormone analogs, leuprolide), non-steroidal antiandrogens *(e.g.,* flutamide), epidermal growth factor inhibitors *(e.g.,* erlotinib, lapatinib, gefitinib) antibodies (e.g., trastuzumab), irinotecan and other agents such as leucovorin. For the treatment of pancreatic cancer, in particular metastatic pancreatic cancer, chemotherapeutic agents or chemotherapy regimens for administration with bevacizumab include gemcitabine and erlotinib and combinations thereof (see also the appended illustrative example herein provided).

In the context of the present disclosure, "homology" with reference to an amino acid sequence is understood to refer to a sequence identity of at least 80%, particularly an identity of at least 85%, at least 90% or at least 95% over the full length of the sequence as defined by the SEQ ID NOs provided herein. In the context of this disclosure, a skilled person would understand that homology covers further allelic variation(s) of the marker/indicator proteins in different populations and ethnic groups.

As used herein, the term "polypeptide" relates to a peptide, a protein, an oligopeptide or a polypeptide which encompasses amino acid chains of a given length, wherein the amino acid residues are linked by covalent peptide bonds. However, peptidomimetics of such proteins/polypeptides are also encompassed by the disclosure wherein amino acid(s) and/or peptide bond(s) have been replaced by functional analogs, e.g., an amino acid residue other than one of the 20 gene-encoded amino acids, e.g., selenocysteine. Peptides, oligopeptides and proteins may be termed polypeptides. The terms polypeptide and protein are used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in a voluminous research literature. The term polypeptide also refers to and encompasses the term "antibody" as used herein.

The terms "treating" and "treatment" as used herein refer to remediation of, improvement of, lessening of the severity of, or reduction in the time course of the disease or any parameter or symptom thereof. Preferably said patient is a human patient and the disease to be treated is pancreatic cancer, in particular metastatic pancreatic cancer.

The terms "assessing" or "assessment" of such a patient relates to methods of determining the expression levels of one or more of the marker/indicator proteins described herein, including VEGFA, VEGFR2 and PLGF, and/or for selecting such patients based on the expression levels of such marker/indicator proteins relative to control levels established in patients diagnosed with pancreatic cancer, in particular metastatic pancreatic cancer.

The term "expression level" as used herein refers may also refer to the concentration or amount of marker/indicator proteins of the present disclosure in a sample.

In addition to the methods described above, the disclosure also encompasses further immunoassay methods for assessing or determining the expression level of one or more of VEGFA, VEGFR2 and PLGF, such as by Western blotting and ELISA-based detection. As is understood in the art, the expression level of the marker/indicator proteins of the disclosure may also be assessed at the mRNA level by any suitable method known in the art, such as Northern blotting, real time PCR, and RT PCR. Immunoassay- and mRNA-based detection methods and systems are well known in the art and can be deduced from standard textbooks, such as Lottspeich (Bioanalytik, Spektrum Akademisher Verlag, 1998) or Sambrook and Russell (Molecular Cloning: A Laboratory Manual, CSH Press, Cold Spring Harbor, NY, U.S.A., 2001). The described methods are of particular use for determining the expression levels of VEGFA, VEGFR2 and PLGF in a patient or group of patients relative to control levels established in a population diagnosed with pancreatic cancer, in particular metastatic pancreatic cancer.

The expression level of one or more of VEGFA, VEGFR2 and PLGF, can also be determined on the protein level by taking advantage of immunoagglutination, immunoprecipitation (e.g., immunodiffusion, immunelectrophoresis, immune fixation), western blotting techniques *(e.g.,* (*in situ*) immuno cytochemistry, affinitychromatography, enzyme immunoassays), and the like. Amounts of purified polypeptide in solution may also be determined by physical methods, *e.g.* photometry. Methods of quantifying a particular polypeptide in a mixture usually rely on specific binding, e.g., of antibodies. Specific detection and quantitation methods exploiting the specificity of antibodies comprise for example immunoassay methods. For example, concentration/amount of marker/indicator proteins of the present disclosure in a patient sample may be determined by enzyme linked-immunosorbent assay (ELISA). Alternatively, Western Blot analysis or immunostaining can be performed. Western blotting combines separation of a mixture of proteins by electrophoresis and specific detection with antibodies. Electrophoresis may be multidimensional such as 2D electrophoresis. Usually, polypeptides are separated in 2D electrophoresis by their apparent molecular weight along one dimension and by their isoelectric point along the other direction.

As mentioned above, the expression level of the marker/indicator proteins according to the present disclosure may also be reflected in an increased expression of the corresponding gene(s) encoding the VEGFA, VEGFR2 and/or PLGF. Therefore, a quantitative assessment of the gene product prior to translation (e.g. spliced, unspliced or partially spliced mRNA) can be performed in order to evaluate the expression of the corresponding gene(s). The person skilled in the art is aware of standard methods to be used in this context or may deduce these methods from standard textbooks (e.g. Sambrook, 2001, loc. cit.). For example, quantitative data on the respective concentration/amounts of mRNA encoding one or more of VEGFA, VEGFR2 and/or PLGF can be obtained by Northern Blot, Real Time PCR and the like.

In a further aspect of the disclosure, the kit of the disclosure may advantageously be used for carrying out a method of the disclosure and could be, inter alia, employed in a variety of applications, e.g., in the diagnostic field or as a research tool. The parts of the kit of the disclosure can be packaged individually in vials or in combination in containers or multicontainer units. Manufacture of the kit follows preferably standard procedures which are known to the person skilled in the art. The kit or diagnostic compositions may be used for detection of the expression level of one or more of VEGFA, VEGFR2 and PLGF in accordance with the herein-described methods of the disclosure, employing, for example, immunohistochemical techniques described herein.

Although exemplified by the use of bevacizumab, the disclosure encompasses the use of other angiogenesis inhibitors as known in the art for use in combination with standard chemotherapy regimens. The terms "angiogenesis inhibitor" as used herein refers to all agents that alter angiogenesis (e.g. the process of forming blood vessels) and includes agents that block the formation of and/or halt or slow the growth of blood vessels. Nonlimiting examples of angiogenesis inhibitors include, in addition to bevacizumab, pegaptanib, sunitinib, sorafenib and vatalanib. Preferably, the angiogenesis inhibitor for use in accordance with the methods of the present invention is bevacizumab. As used herein, the term "bevacizumab" encompass all corresponding anti-VEGF antibodies or anti-VEGF antibody fragments, that fulfil the requirements necessary for obtaining a marketing authorization as an identical or biosimilar product in a country or territory selected from the group of countries consisting of the USA, Europe and Japan.

For use in the detection methods described herein, the skilled person has the ability to label the polypeptides, for example antibodies, or oligonucleotides encompassed by the present disclosure. As routinely practiced in the art, hybridization probes for use in detecting mRNA levels and/or antibodies or antibody fragments for use in immunoassay methods can be labelled and visualized according to standard methods known in the art, nonlimiting examples of commonly used systems include the use of radiolabels, enzyme labels, fluorescent tags, biotin-avidin complexes, chemiluminescence, and the like.

The person skilled in the art, for example the attending physician, is readily in a position to administer the bevacizumab in combination with a chemotherapy regimen to the patient/patient group as selected and defined herein. In certain contexts, the attending physician may modify, change or amend the administration schemes for the bevacizumab and the chemotherapy regimen in accordance with his/her professional experience. Therefore, in certain aspects of the present disclosure, a method is provided for the treatment or improving the overall survival and/or progression-free survival of a patient suffering from or suspected to suffer from pancreatic cancer, in particular metastatic pancreatic cancer, with bevacizumab in combination with a chemotherapy regimen, whereby said patient/patient group is characterized in the assessment of a biological sample from the patient (in particular a blood plasma sample), said sample exhibiting an increased expression level of one or more of VEGFA, VEGFR2 and PLGF relative to control levels established in patients diagnosed with pancreatic cancer, in particular metastatic pancreatic cancer. The present disclosure also provides for the use of bevacizumab in the preparation of pharmaceutical composition for the treatment of a patient suffering from or suspected to suffer from pancreatic cancer, in particular metastatic pancreatic cancer, wherein the patients are selected or characterized by the herein disclosed protein marker/indicator status *(i.e.,* one or more of an increased expression level of VEGFA, VEGFR2 and PLGF relative to control levels established in patients diagnosed with pancreatic cancer, in particular metastatic pancreatic cancer.

The figures show:

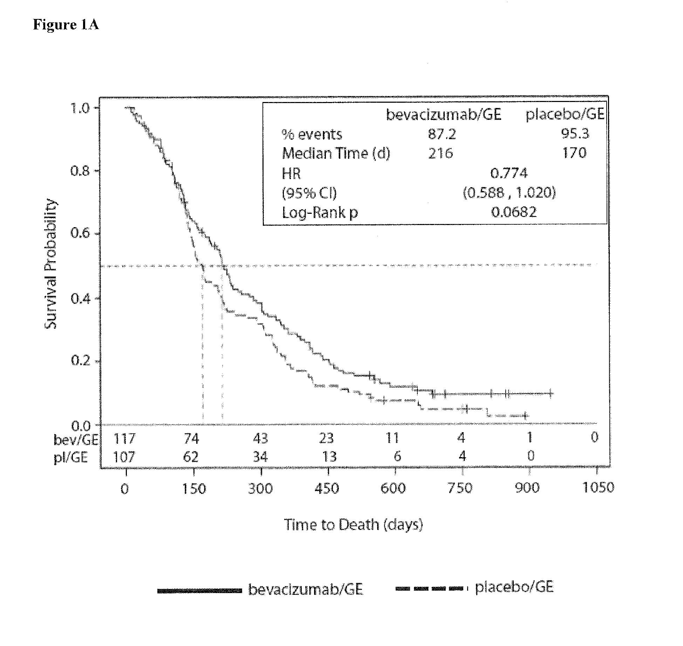

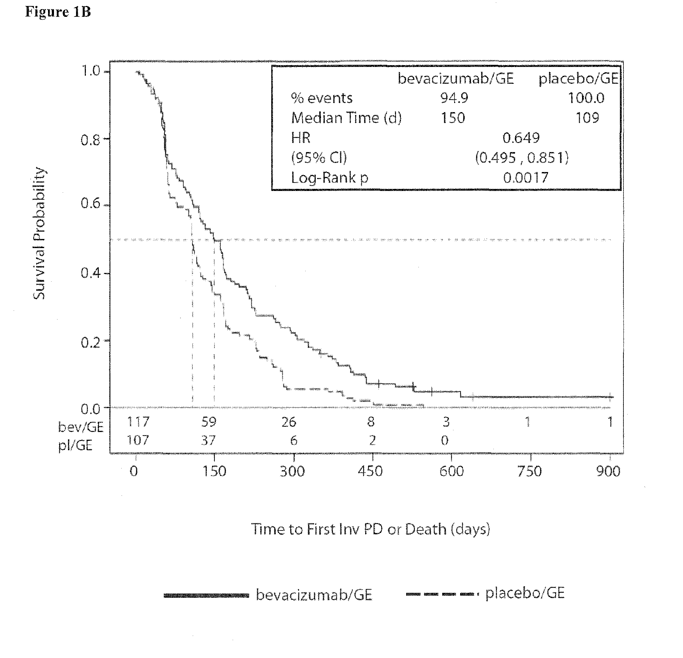

Figure 1: Kaplan Meier Curves for Overall Survival (Figure 1A) and for Progression Free Survival (Figure 1B) for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

Figure 2: Kaplan Meier Curves for association with treatment effect on Overall Survival for the marker VEGFA (Figure 2A) and for association with treatment effect on Progression free survival for the marker VEGFA (Figure 2B), for both high ($\geq$152.9 pg/ml) and low (< 152.9 pg/ml) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

Figure 3: Kaplan Meier Curves for association with treatment effect on Overall Survival for the marker VEGFR2, for both high ($\geq$9.9 ng/ml) and low (< 9.9 ng/ml) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

Figure 4: Kaplan Meier Curves for association with treatment effect on Progression Free Survival for the marker PLGF, for both optimized high ($\geq$36.5 pg/ml) and low (<36.5 pg/ml) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 5:** Kaplan Meier Curves for association with treatment effect on Overall Survival for the markers VEGFA and VEGFR2 (Figure 5A), as a combined expression level for both high (Formula 1 ≥ -0.1) and low (Formula 1 < -0.1) expression levels, and VEGFA and PLGF (Figure 5B), as a combined expression level for both high (Formula 2 ≥ -0.042) and low (Formula 2 < -0.042) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 6:** Kaplan Meier Curves for association with treatment effect on Progression Free Survival for the markers VEGFA and VEGFR2 (Figure 6A), as a combined expression level for both high (Formula 1 ≥ -0.1) and low (Formula 1 < -0.1) expression levels, and VEGFA and PLGF (Figure 6B), as a combined expression level for both high (Formula 2 ≥ -0.042) and low (Formula 2 < -0.042) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 7:** Kaplan Meier Curve for association with treatment effect on Overall Survival for the markers for the markers VEGFA, VEGFR2 and PLGF (Figure 7A), as a combined expression level for both high (Formula 3 ≥ 0.837) and low (Formula 3 < 0.837) expression levels, and for association with treatment effect on Progression Free Survival for the makers VEGFA, VEGFR2 and PLGF (Figure 7B), as a combined expression level for both high (Formula 3 ≥ 0.837) and low (Formula 3 < 0.837) expression levels, for bevacizumab plus gemcitabine-erlotinib therapy versus control placebo plus gemcitabine-erlotinib therapy for patients being treated for metastatic pancreatic cancer.. In the figure, the solid line represents bevacizumab/gemcitabine-erlotinib treatment and the dashed line represents placebo/gemcitabine-erlotinib treatment.

**Figure 8:** SEQ ID NO:1, Exemplary amino acid sequence of VEGFA.

**Figure 9:** SEQ ID NO:2, Exemplary amino acid sequence of VEGFR2.

**Figure 10:** SEQ ID NO:3, Exemplary amino acid sequence of PLGF.

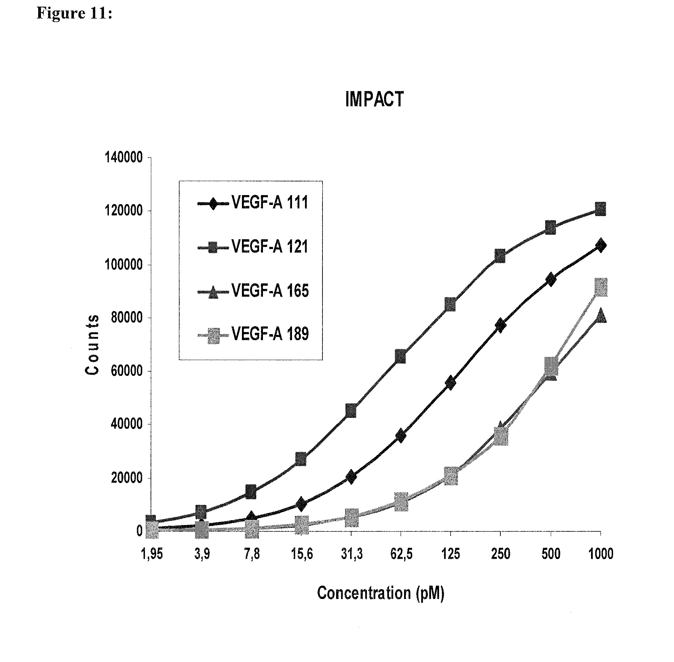

**Figure 11:** Measurements of increasing concentrations of $VEGF_{111}$, $VEGF_{121}$, $VEGF_{165}$ and $VEGF_{189}$ as measured on an IMPACT chip.

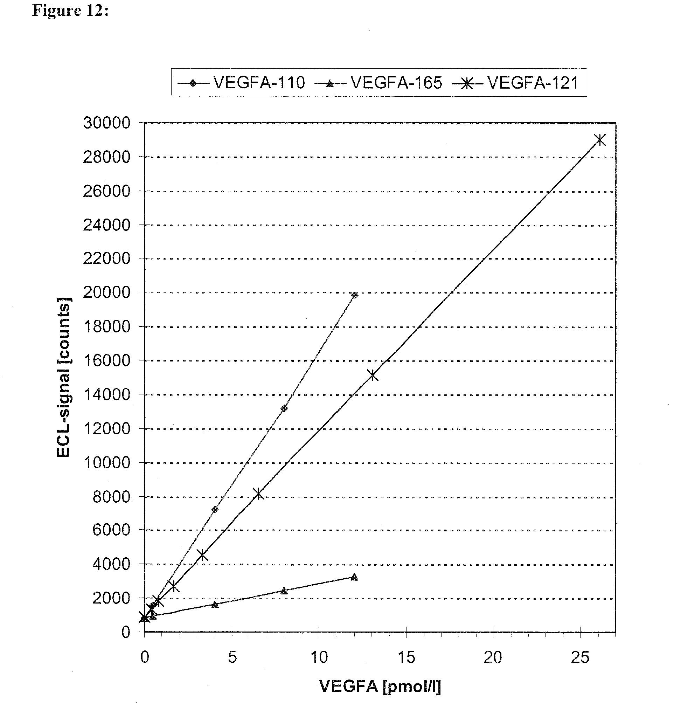

**Figure 12:** Measurements of increasing concentrations of $VEGF_{110}$, $VEGF_{121}$, and $VEGF_{165}$ as measured using the Elecsys® Assay on the automated Elecsys® analyzer.

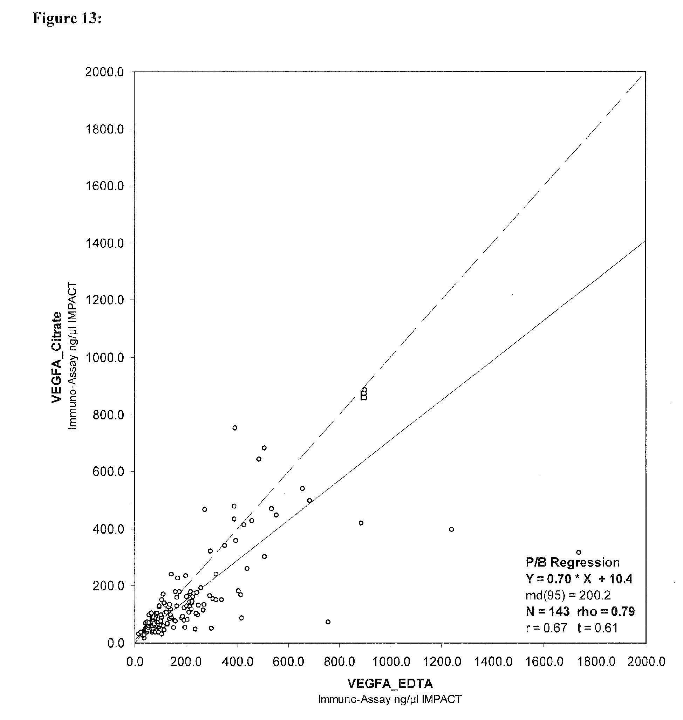

**Figure 13:** Data from EDTA- and Citrate samples from the same patients measured twice with the IMPACT assay. The VEGFA concentration is about 40% higher for EDTA-plasma than for Citrate with a Spearman correlation for the EDTA-Citrate method comparison of about 0.8.

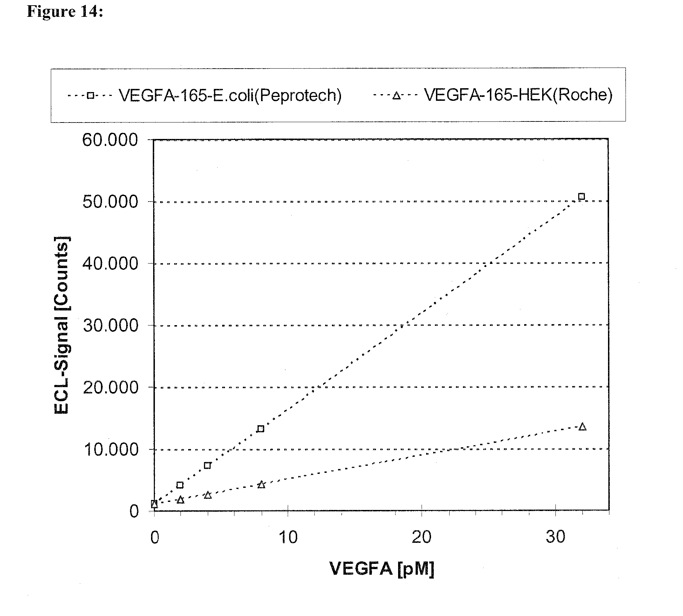

**Figure 14:** Shown are the counts (ECL-signal) measured when increasing concentrations of $VEGF_{165}$, produced recombinantly in E. coli or in HEK-cells, respectively, were measured on the automated Elecsys® analyzer.

The present invention is further illustrated by the following non-limiting illustrative example.

**EXAMPLE 1**

Patients with metastatic pancreatic adenocarcinoma were randomized to gemicitamibe-erlotinib plus bevacizumab (n=306) or placebo (n=301).

Blood plasma samples were collected from patients participating in a randomized phase-III study comparing the results of adding bevacizumab to gemicitamibe-erlotinib therapy for the treatment of metastatic pancreatic cancer (the BO17706 study, *see* Figure 1, also see Van Cutsem, J Clin. Oncol. 2009 27:2231-2237). Patients with metastatic pancreatic adenocarcinoma were randomized to gemicitamibe-erlotinib plus bevacizumab (n=306) or placebo (n=301). Patients with metastatic pancreatic adenocarcinoma were randomly assigned to receive gemcitabine (1,000

mg/m$^2$/week), erlotinib (100 mg/day), and bevacizumab (5 mg/kg every 2 weeks) or gemcitabine, erlotinib, and placebo.

An investigation of the status of biomarkers related to angiogenesis and tumorigenesis revealed that the expression levels of three biomarkers relative to control levels determined in the entire biomarker patient population correlated with an improved treatment parameter. In particular, patients exhibiting a higher expression level of VEGFA relative to control levels determined in the entire biomarker patient population, demonstrated a prolonged overall survival and a prolonged progression free survival in response to the addition of bevacizumab to gemicitamibe-erlotinib therapy. Patients exhibiting a higher expression level of VEGFR2 relative to control levels determined in the entire biomarker patient population, demonstrated a prolonged overall survival in response to the addition of bevacizumab to gemicitamibe-erlotinib therapy. Patient exhibiting a higher expression level of PLGF relative to control levels determined in the entire biomarker patient population, demonstrated a prolonged progression free survival in response to the addition of bevacizumab to gemicitamibe-erlotinib therapy. Also patients exhibiting higher combined expression level of VEGFA and VEGFR2 relative to control levels determined in the entire biomarker patient population, demonstrated a prolonged overall survival and a prolonged progression free survival in response to the addition of bevacizumab to gemicitamibe-erlotinib therapy. In addition, patients exhibiting higher combined expression level of VEGFA and PLGF relative to control levels determined in the entire patient population, demonstrated a prolonged overall survival and a prolonged progression free survival in response to the addition of bevacizumab to gemcitamibe-erlotinib therapy. Patients exhibiting higher combined expression level of VEGFA, VEGFR2 and PLGF relative to control levels determined in the entire patient population, demonstrated a prolonged overall survival and a prolonged progression free survival in response to the addition of bevacizumab to gemcitamibe-erlotinib therapy

**Patients and Immunochemical Methods**

A total of 607 patients participated in the BO17706 study, and blood plasma samples from 224 of the participants were available for biomarker analysis. The baseline characteristics of the 224 patients in the biomarker analysis are provided in Table 1.

*Table 1. Baseline characteristics: biomarker population (n=224)*

|  | bevacizumab | | placebo | |
|---|---|---|---|---|
|  | N | (%) | N | (%) |
| Sex |  |  |  |  |
| Female | 45 | 38.46 | 32 | 29.91 |
| Male | 72 | 61.54 | 75 | 70.09 |
|  |  |  |  |  |
| Age Category (years) |  |  |  |  |
| <65 | 73 | 62.39 | 71 | 66.36 |
| > = 65 | 44 | 37.61 | 36 | 33.64 |
|  |  |  |  |  |
| KPS (%) Category at Baseline |  |  |  |  |
| <80% | 15 | 12.82 | 13 | 12.15 |
| >=80% | 102 | 87.18 | 94 | 87.85 |
|  |  |  |  |  |
| VAS Category at Baseline |  |  |  |  |
| below baseline (not available) | 10 | 8.55 | 16 | 14.95 |
| <20 | 68 | 58.12 | 56 | 52.34 |
| >=20 | 39 | 33.33 | 35 | 32.71 |
|  |  |  |  |  |
| CRP Category (median value) at Baseline (mg/dL) |  |  |  |  |
| below baseline (not available) | 13 | 11.11 | 9 | 8.41 |

(continued)

|  | bevacizumab | | placebo | |
|---|---|---|---|---|
|  | N | (%) | N | (%) |
| <=1.4 | 52 | 44.44 | 49 | 45.79 |
| >1.4 | 52 | 44.44 | 49 | 45.79 |
| VAS: Visual Analogue Scale of Pain<br>KPS: Karnofsky Performance Score | | | | |

**Blood Plasma Analysis**

Plasma samples were collected after randomization and before any study treatment was given to the patients and VEGFA, vascular endothelial growth factor receptor 1 (VEGFR1), VEGFR2, PLGF and E-SELECTIN were measured using a multiplex ELISA assay (Impact) from Roche Diagnostics GmbH.

IMPACT Multiplex Assay Technology

Roche Professional Diagnostics (Roche Diagnostics GmbH) has developed a multimarker platform under the working name IMPACT (Immunological MultiParameter Chip Technology). The technology is based on a small polystyrene chip manufactured by procedures as disclosed in EP 0939319 and EP 1610129. The chip surface was coated with a streptavidin layer, onto which the biotinylated antibodies were then spotted for every assay. For each marker, spots of antibodies were loaded in a vertical line onto the chip. During the assay, the array was probed with specimen samples containing the specific analytes.

The plasma volume required per specimen for measuring all markers on one chip was 8 $\mu$L, which was applied together with 32 $\mu$L of an incubation buffer (50 mM HEPES pH 7.2, 150 mM NaCl, 0.1 % Thesit, 0.5% bovine serum albumin and 0.1% Oxypyrion as a preservative agent). After incubation for 12 minutes and washing of the chip using a washing buffer (5 mM Tris pH 7.9, 0.01% Thesit and 0.001% Oxypyrion) the digoxigenylated monoclonal antibody mix was added (40 $\mu$L of incubation buffer including a mix of the analyte-specific antibodies labeled with Digoxigenin) and was incubated for an additional 6 minutes to bind onto the captured analytes. The second antibody was finally detected with 40 $\mu$L of a reagent buffer (62.5 mM TAPS pH 8.7, 1.25 M NaCl, 0.5% bovine serum albumin, 0.063% Tween 20 and 0.1% Oxypyrion) including an anti-digoxigenin antibody conjugate coupled with fluorescent latex. Using this label, 10 individual binding events in a single spot could be detected, resulting in very high sensitivity down to the fmol/L concentration. Chips were transported into the detection unit, and a charge coupled device (CCD) camera generated an image that was transformed into signal intensities using dedicated software. Individual spots were automatically located at predefined positions and quantified by image analysis. For each marker, lines of 10-12 spots were loaded on the chips, and a minimum of 5 spots was required to determine the mean concentration of samples. The advantages of the technology are the ability of multiplexing up to 10 parameters in a sandwich or competitive format. The calibrators and patient samples were measured in duplicate. One run was designed to contain a total of 100 determinations, including 2 multi-controls as a run control. Since some of the selected analytes react with each other (i.e VEGFA and PLGF with VEGFR1 or VEGRF2 or VEGFA forms heterodimers with PLGF), the 5 analytes were divided on three different chips as follows:

Chip 1: VEGFA
Chip 2: VEGFR1, VEGFR2, E-Selectin
Chip 3: PLGF

The following antibodies were used for the different assays:

| Analyte | Capture antibody | Manufacturer | Detection antibody | Manufacturer |
|---|---|---|---|---|
| VEGFA | <VEGF-A>M-3C5 | Bender RELIATech | <VEGF>M-26503 | R&D Systems |
| VEGFR1 | <VEGF-R1>M-49560 | Roche Diagnostics | <VEGF-R1>M-49543 | Roche Diagnostics |
| VEGFR2 | <VEGF-R2>M-89115 | R&D Systems | <VEGF-R2>M-89109 | R&D Systems |
| E-Selectin | <E-Selectin>M-BBIG-E5 | R&D Systems | <E-Selectin>M-5D11 | R&D Systems |

(continued)

| Analyte | Capture antibody | Manufacturer | Detection antibody | Manufacturer |
|---|---|---|---|---|
| PLGF | <PLGF>M-2D6D5 | Roche Diagnostics | <PLGF>M-6A11D2 | Roche Diagnostics |

## Statistical Analysis

Sample median was used to dichotomize biomarker values as low (below median) or high (above median).

Hazard Ratio of treatment effect in sub-group of patients with high or low biomarker levels were estimated with proportional hazard cox regression analysis.

In addition, proportional hazard cox regressions was used to evaluate the association between biomarker level and treatment effect. The model included the following covariates: trial treatment, biomarker level, interaction term of treatment by biomarker level. Wald test for the interaction term was used to determined the association between biomarker level and treatment effect. P-value below 0.05 was considered significant.

## Results

Blood Plasma Markers

The baseline descriptive statistics of the biomarkers are presented in Table 2.

*Table 2: Descriptive Statistics of Biomarker Values (Baseline)*

| | *VEGFA* (pg/ml) at baseline | *VEGFR2* (ng/mL) at baseline | *PIGF* (pg/ml) at baseline |
|---|---|---|---|
| *min* | 3.06 | 0.23 | 0 |
| *qu 25%* | 80.08 | 7.9 | 32.9 |
| *median* | 152.80 | 9.9 | 37.8 |
| *qu 75%* | 275.90 | 12.6 | 43.6 |
| *max* | 2127.00 | 58.1 | 142.3 |
| *mean* | 215.30 | 10.4 | 39.4 |
| *sd* | 254.8 | 4.7 | 12.5 |

Table 3 presents the univariate analysis of the association of the selected biomarkers with treatment effect on overall survival.

*Table 3: Association with treatment effect on Overall Survival - (uni-variate analysis)*

| | HR (95% CI) | P-value for interaction |
|---|---|---|
| VEGFA low | 1.018 (0.69, 1.5) | 0.0308 |
| VEGFA high | 0.558 (0.37,0.83) | |
| VEGFR2 low | 1.057 (0.72,1.55) | 0.0461 |
| VEGFR2 high | 0.583 (0.39,0.87) | |
| PLGF low | 1.048 (0.67, 1.63) | 0.089 |
| PLGF high | 0.659 (0.46, 0.95) | |

In this analysis, for VEGFA, Low VEGFA <152.9 pg/ml and High VEGFA $\geq$ 152.9 pg/ml, for VEGFR2, Low VEGFR2 < 9.9 ng/ml and High VEGFRA $\geq$ 9.9 ng/ml, and for PLGF, Low PLGF < 36.5 pg/ml and High PLGF $\geq$ 36.5 pg/ml, were used.

For VEGFA and VEGFR2 the cut-off levels were determined as sample data median value, such that 50% of patients have high expression and 50% of patients have low expression, as per pre-determined analysis plan. The PLGF cut-off levels were determined as 42nd percentile of the data. Accordingly, 58% of patients have high expression of PLGF and 42% have low expression. The cut-off was determined in order to increase the statistical difference between treatment effect in high and low level subgroup.

This result table shows that the Hazard Ratio for treatment effect is significantly better in the subset of patients with high VEGFA compared to patients with low VEGFA. This result table also shows that the Hazard Ratio for treatment effect is significantly better in the subset of patients with high VEGFR2 compared to patients with low VEGFR2. Therefore, VEGFA and VEGFR2 are each independent predictive biomarkers for Bevacizumab treatment effect on overall survival.

Table 4 presents the univariate analysis of the association of the selected biomarkers with treatment effect on progression free survival.

Table 4: Association with treatment effect on Progression Free Survival (univariate analysis)

| | HR (95% CI) | P-value for interaction |
|---|---|---|
| VEGFA low | 0.771 (0.53,1.13) | 0.0603 |
| VEGFA high | 0.522 (0.35,0.78) | |
| VEGFR2 low | 0.773 (0.53,1.12) | 0.4012 |
| VEGFR2 high | 0.541 (0.36,0.81) | |
| PLGF low | 0.957 (0.63,1.46) | 0.0136 |
| PLGF high | 0.505 (0.35,0.73) | |

In this analysis, for VEGFA, Low VEGFA < 152.9 pg/ml and High VEGFA $\geq$ 152.9 pg/ml, for VEGFR2, Low VEGFR2 < 9.9 ng/ml and High VEGFRA $\geq$ 9.9 ng/ml, and for PLGF, Low PLGF < 36.5 pg/ml and High PLGF $\geq$ 36.5 pg/ml, were used. For VEGFA and VEGFR2 the cut-off levels were determined as sample data median value, such that 50% of patients have high expression and 50% of patients have low expression, as per pre-determined analysis plan. The PLGF cut-off levels were determined as 42nd percentile of the data. Accordingly, 58% of patients have high expression of PLGF and 42% have low expression. The cut-off was determined in order to increase the statistical difference between treatment effect in high and low level subgroup.

This result table shows that the Hazard Ratio for treatment effect is significantly better in the subset of patients with high VEGFA compared to patients with low VEGFA. This result table also shows that the Hazard Ratio for treatment effect is significantly better in the subset of patients with high PLGF compared to patients with low PLGF. Therefore, VEGFA and PLGF are each independent predictive biomarkers for bevacizumab treatment effect on progression free survival.

Table 5 presents the analysis of biomarker combinations association with treatment effect on overall survival.

For this analysis the following equations were used:

$$\text{Formula 1: norm(VEGFA)} + 1.3 * \text{norm(VEGFR2). Cut-point} = \text{median or } 0$$

Equivalent formula: VEGFA+3.3*VEGFR2. Cut-point= median or 0
and

$$\text{Formula 2: } 0.25 * \text{norm(VEGFA)} + 0.21 * \text{norm(PLGF), cut-point=median or } 0$$

Equivalent formula: 0.19*VEGFA+0.67*PLGF, cut-point= median or 4.8

Where we use log2 transformation and

$$x_i \rightarrow norm(x_i) = \frac{\log 2(x_i) - median(\log 2(x))}{mad(\log 2(x))}$$

*Table 5: Association with treatment effect on Overall Survival (bi-marker analysis)*

|  | HR (95% CI) | P-value for interaction |
|---|---|---|
| VEGFA & VEGFR2 low | 1.317 (0.89,1.94) | 0.0002 |
| VEGFA & VEGFR2 high | 0.42 (0.28,0.64) | |
| VEGFA & PLGF low | 1.101 (0.74,1.64) | 0.0096 |
| VEGFA & PLGF high | 0.546 (0.37,0.81) | |

In this analysis, a high combined expression level of VEGFA and VEGFR2 is (Formula 1 ≥ -0.10) and a low combined expression of VEGFA and VEGFR2 is (Formula 1 < -0.10), and a high combined expression level of VEGFA and PLGF is (Formula 2 ≥ -0.042) and a low combined expression of VEGFA and PLGF is (Formula 2 < -0.042).

This results table shows that the Hazard Ratio for treatment effect is significantly better in the subset of patients with high VEGFA & VEGFR2 combination compared to patients with low VEGFA & VEGFR2 combination. This result table also shows that the Hazard Ratio for treatment effect is significantly better in the subset of patients with high VEGA & PLGF combination compared to patients with low VEGFA & PLGF combination. Therefore, VEGFA & VEGFR2 combination and VEGFA & PLGF combination are each independent predictive biomarkers for bevacizumab treatment effect on overall survival.

Table 6 presents the analysis of biomarker combinations association with treatment effect on progression free survival.

For this analysis the following equations were used:

$$\text{Formula 1: } norm(VEGFA)+1.3*norm(VEGFR2). \text{ Cut-point= median or } 0$$

Equivalent formula:

$$VEGFA+3.3*VEGFR2.$$

Cut-point= median or 0
and

$$\text{Formula 2: } 0.25*norm(VEGFA)+0.21*norm(PLGF), \text{ cut-point=median or } 0$$

Equivalent formula:

$$0.19*VEGFA+0.67*PLGF,$$

cut-point= median or 4.8

Where we use log2 transformation and

$$x_i \rightarrow norm(x_i) = \frac{\log 2(x_i) - median(\log 2(x))}{mad(\log 2(x))}$$

*Table 6: Association with treatment effect on Progression Free Survival (bi-marker analysis)*

|  | HR (95% CI) | P-value for interaction |
|---|---|---|
| VEGFA & VEGFR2 low | 0.984 (0.68,1.43) | 0.0040 |
| VEGFA & VEGFR2 high | 0.411 (0.26,0.64) | |

(continued)

|  | HR (95% CI) | P-value for interaction |
|---|---|---|
| VEGFA & PLGF low | 0.936 (0.64,1.37) | 0.0011 |
| VEGFA & PLGF high | 0.426 (0.28,0.64) | |

In this analysis, a high combined expression level of VEGFA and VEGFR2 is (Formula 1 ≥ -0.10) and a low combined expression of VEGFA and VEGFR2 is (Formula 1 < -0.10), and a high combined expression level of VEGFA and PLGF is (Formula 2 ≥ -0.042) and a low combined expression of VEGFA and PLGF is (Formula 2 < -0.042).

This results table shows that the Hazard Ratio for treatment effect is significantly better in the subset of patients with high VEGFA & VEGFR2 combination compared to patients with low VEGFA & VEGFR2 combination. This result table also shows that the Hazard Ratio for treatment effect is significantly better in the subset of patients with high VEGA & PLGF combination compared to patients with low VEGFA & PLGF combination. Therefore, VEGFA & VEGFR2 combination and VEGFA & PLGF combination are each independent predictive biomarkers for bevacizumab treatment effect on progression free survival. Tables 7 and Table 8 present the analysis of biomarker combinations of VEGFA, VEGFR2 and PLGF association with treatment effect on overall survival and progression free survival, respectively.

In this analysis, the following equation was used:

$$\text{Formula 3: } 0.0127 * \ln(PLGF+1) + 0.144 * \ln(VEGFR2+1) + 0.0949 * \ln(VEGFA + 1)$$

Where ln = log basis e

*Table 7: Association with treatment effect on Overall Survival (tri-marker analysis)*

| Overall Survival | HR (95% CI) | P-value for interaction |
|---|---|---|
| VEGFA & VEGFR2 & PLGF low | 1.051 (0.71,1.55) | 0.0033 |
| VEGFA & VEGFR2 & PLGF high | 0.554 (0.38, 0.8) | |

*Table 8: Association with treatment effect on Progression Free Survival (tri-marker analysis)*

| Progression Free Survival | HR (95% CI) | P-value for interaction |
|---|---|---|
| VEGFA & VEGFR2 & PLGF low | 0.974 (0.64,1.48) | 0.0096 |
| VEGFA & VEGFR2 & PLGF high | 0.488 (0.34,0.71) | |

In this analysis, for overall survival, a high combined expression level of VEGFA, VEGFR2 and PLGF is (Formula 3 ≥ 0.837) and a low combined expression of VEGFA, VEGFR2 and PLGF is (Formula 3 < 0.837), and for progression free survival, a high combined expression level of VEGFA, VEGFR2 and PLGF is (Formula 3 ≥ 0.837) and a low combined expression of VEGFA, VEGFR2 and PLGF is (Formula 3 < 0.837).

This results table shows that the Hazard Ratio for treatment effect is significantly better in the subset of patients with high VEGFA & VEGFR2 & PLGF combination compared to patients with low VEGFA & VEGFR2 & PLGF combination. Therefore, VEGFA & VEGFR2 & PLGF combination is a predictive biomarkers for bevacizumab treatment effect on progression free survival.

This results table also shows that for overall survival the Hazard Ratio for treatment effect is significantly better in the subset of patients with high VEGFA & VEGFR2 & PLGF combination compared to patients with low VEGFA & VEGFR2 & PLGF combination. Therefore, VEGFA & VEGFR2 & PLGF combination is a predictive biomarkers for Bevacizumab treatment effect on overall survival.

Example 2: Detection of shorter isoforms of VEGF-A using the IMPACT Assay

This example demonstrates that, based on the antibodies used for detection of VEGF-A on the IMPACT platform, the shorter isoforms of VEGF-A are preferentially measured as compared to the longer isoforms of VEGF-A.

The assay was performed as described above under the section relating to the IMPACT technology using the antibodies listed in the table before the "statistical analysis" section.

Four different VEGF-A forms, i.e. $VEGF_{111}$, $VEGF_{121}$, $VEGF_{165}$ and $VEGF_{189}$ were available and used in the analysis. $VEGF_{111}$, $VEGF_{121}$ (both derived from expression in E. coli), and $VEGF_{165}$ (obtained recombinantly in an insect cell line) was purchased from R&D Systems, Minneapolis, USA and $VEGF_{189}$ was obtained from RELIATech, Wolfenbüttel, Germany. It has turned out later that VEGF189 appears to be rather unstable and that the data obtained with that material cannot be relied upon. As shown in Figure 11 the shorter isoforms having 111 or 121 amino acids, respectively, which had been produced in E. coli and are not secondarily modified, e.g., not glycosylated, are detected better as compared to the longer isoforms with 165 amino acids. VEGF165 had been obtained in an insect cell line and is at least partially glycosylated. The biologically interesting plasmin cleavage product $VEGF_{110}$ was not available for testing at this point in time, but is has to be expected that detection of this isoform will be comparable to what is seen for the VEGF-molecule with 111 amino acids.

## Example 3: Detection of short VEGF isoforms using the Elecsys® Analyzer

This example describes experiments demonstrating that an assay using the Elecsys® Analyzer and a corresponding assay can be used to detect short VEGF isoforms in human plasma.

The VEGF-A assay was transferred from IMPACT to the automated in-vitro diagnostics system Elecsys® (Roche Diagnostics GmbH, Mannheim). The same capture antibody as in the IMPACT Assay, <hVEGF-A>-m3C5 (RELIATech, Wolfenbüttel) was used, while the capture antibody <hVEGF-A>-m25603 (R&D Systems, Minneapolis) used on the IMPACT system was replaced by <hVEGF-A>-mA4.6.1 (Genentech, South San Francisco).

The immunoassays running on the automated Elecsys® system are immuno assays using electrochemiluminescense (ECLIA) as the signal generating technology. In the present sandwich assay the biotinylated capture antibody binds to streptavidin coated, magnetic microparticles and the ruthenylated detection antibody allows for signal generation. 75 μl of biotinylated <VEGF-A>-m3C5 at 1.5 μg/ml and 75 μl of ruthenylated <VEGF-A>M-A.4.6.1 at 2 μg/ml both in reaction buffer (50 mM Tris (pH 7.4), 2 m M EDTA, 0.1 % thesit, 0.2 % bovine IgG, 1.0 % bovine serum albumin) were incubated for 9 minutes with 20 μl of sample. 30 μl of a microparticle suspension was added after the first 9 minutes of incubation and the whole mixture then incubated for an additional 9 minutes. During these incubation steps an antibody analyte antibody sandwich is formed that is bound to the microparticles. Finally the microparticles were transferred to the detection chamber of the Elecsys system for signal generation and readout.

The cleavage product/isoform preference of the Elecsys® VEGF-A assay was assessed with purified recombinant proteins: VEGF 110 (produced by plasmin cleavage at Genentech, South San Francisco), VEGF 121 and VEGF 165 (both produced in an insect cell line and supplied by R&D Systems, Minneapolis). The preferential binding of short VEGF isoforms that had been seen with the IMPACT® Assay was confirmed in the Elecsys assay. As shown in Figure 12, in the Elecsys® assay the isoforms VEGF 121 and the plasmin cleavage product VEGF 110, respectively, both were detected with an approximately 5-fold higher sensitivity than VEGF 165.

## Example 4: Detection of short VEGF isoforms in plasma collected in Na citrate and EDTA

Paired plasma samples were collected from patients with HER2+ locally recurrent or metastatic breast cancer in both an EDTA monovette (5mL)- and Citrate Monovette collection tube (5mL). Within 30 minutes of blood collection, blood tubes were placed into the centrifuge and spun 1500 g at room temperature for 10 minutes, until cells and plasma were separated. Immediately after centrifugation, the plasma was carefully transferred into a propylene transfer tube and then aliquotted equally into 2 storage tubes (half volume each approximately 1.25 mL) using a pipette. The levels of VEGF-A in the samples were measured using the IMPACT Assay described above. As shown in Figure 13, the VEGFA concentration is about 40% higher for plasma samples collected and stored in EDTA compared to plasma samples collected and stored in citrate with a Spearman correlation for the EDTA-Citrate MC of about 0.8 for baseline samples collected prior to treatment.

## Example 5: Comparative measurement of unmodified and modified VEGF165 on the Elecsys analyzer

This example describes experiments demonstrating that the Elecsys® Analyzer and a corresponding assay can be used to detect unmodified VEGF in human plasma.

The VEGF-A assay was transferred from IMPACT to the automated in-vitro diagnostics system Elecsys® (Roche Diagnostics GmbH, Mannheim). The same capture antibody as in the IMPACT assay, <hVEGF-A>-m3C5 (RELIATech GmbH, Wolfenbüttel) was used, while the detection antibody <hVEGF-A>-m25603 (R&D Systems, Minneapolis) used on the IMPACT system was replaced by <hVEGF-A>-mA4.6.1 (Genentech, South San Francisco).

The immunoassays running on the automated Elecsys system are immuno assays using electrochemiluminescense (ECLIA) as the signal generating technology. In the present sandwich assay the biotinylated capture antibody binds to streptavidin coated, magnetic microparticles and the ruthenylated detection antibody allows for signal generation.

75 μl of biotinylated <VEGF-A>-m3C5 at 1.5 μg/ml and 75 μl of ruthenylated <VEGF-A>M-A.4.6.1 at 2 μg/ml both in reaction buffer (50 mM Tris (pH 7.4), 2 m M EDTA, 0.1 % thesit, 0.2 % bovine IgG, 1.0 % bovine serum albumin) were incubated for 9 minutes with 20 μl of sample. 30 μl of a microparticle suspension was added after the first 9 minutes of incubation and the whole mixture then incubated for an additional 9 minutes. During these incubation steps an antibody-analyte-antibody sandwich is formed that is bound to the microparticles. Finally the microparticles were transferred to the detection chamber of the Elecsys system for signal generation and readout.

The preference of the Elecsys VEGF-A assay was assessed with purified recombinant proteins: VEGF165 (produced recombinantly in E. coli by Peprotech) and VEGF165 (produced recombinantly in HEK-cells at Roche Diagnostics, Germany). The preferential binding of unmodified VEGF165 that had been seen with the IMPACT assay was confirmed in the Elecsys assay. As shown in Figure 14, in the Elecsys assay the unmodified VEGF 165 was detected with an approximately 5-fold higher sensitivity than modified VEGF 165.

## Claims

1. An in vitro method for the identification of a patient responsive to or sensitive to the addition of bevacizumab treatment to a chemotherapy regimen, said method comprising determining the protein expression level of one or more of VEGFA, VEGFR2 and PLGF in a blood plasma sample from a patient suspected to suffer from or being prone to suffer from pancreatic cancer, whereby an increased expression level of one or more of VEGFA, VEGFR2 and PLGF relative to control expression levels determined in patients suffering from pancreatic cancer is indicative of a sensitivity of the patient to the addition of bevacizumab to said chemotherapy regimen, wherein the pancreatic cancer is metastatic pancreatic cancer and the chemotherapy regimen comprises gemcitabine and erlotinib.

2. An in vitro method of predicting the response to or sensitivity to the addition of bevacizumab to a chemotherapy regimen of a patient suspected to suffer from, suffering from or prone to suffer from pancreatic cancer comprising determining the protein expression level of one or more of VEGFA, VEGFR2 and PLGF in a patient blood plasma sample, wherein the pancreatic cancer is metastatic pancreatic cancer and the chemotherapy regimen comprises gemcitabine and erlotinib.

3. The method of claim 1, wherein the treatment effect is progression-free survival.

4. The method of claim 1, wherein the treatment effect is overall survival.

5. The method of claims 2 to 4, wherein the protein expression level determined is of VEGFA or PLGF.

6. The method of claims 2 to 4 , wherein the protein expression level determined is of VEGFA or VEGFR2.

7. The method of claims 1 to 4, wherein protein expression level determined is a combined expression level of VEGFA and VEGFR2.

8. The method of claims 1 to 4, wherein the protein expression level determined is a combined expression level of VEGFA and PLGF.

9. The method of claims 1 to 4, wherein the protein expression level determined is a combined expression level of VEGFA, VEGFR2 and PLGF.

10. The method of claims 1 to 9, wherein said expression level is detected by an immunoassay method.

11. The method of claim 10, wherein said immunoassay method is ELISA.

12. The method of claims 1 to 11, wherein said patient is being co-treated with one or more anti-cancer therapies.

13. The method of claim 12, wherein said anti-cancer therapy is radiation.

14. The method of claims 1 to 13, wherein said sample is obtained before neoadjuvant or adjuvant therapy or after neoadjuvant or adjuvant therapy.

**Patentansprüche**

1. *In vitro*-Verfahren zur Identifizierung eines Individuums, das auf das Hinzufügen einer Behandlung mit Bevacizumab zu einem Chemotherapie-Behandlungsplan anspricht oder sensibel reagiert, wobei das Verfahren das Bestimmen des Proteinexpressionsspiegels eines oder mehrerer von VEGFA, VEGFR2 und PLGF in einer Blutplasmaprobe eines Individuums umfasst, bei dem der Verdacht besteht oder das eine Disposition hat, an Pankreaskrebs zu leiden, wobei ein erhöhter Expressionsspiegel eines oder mehrerer von VEGFA, VEGFR2 und PLGF, im Verhältnis zu Kontrollexpressionsspiegel, die bei an Pankreaskrebs leidenden Individuen bestimmt werden, auf eine Sensibilität des Individuums auf das Hinzufügen von Bevacizumab zu dem Chemotherapie-Behandlungsplan hinweist, wobei der Pankreaskrebs metastatischer Pankreaskrebs ist und der Chemotherapie-Behandlungsplan Gemcitabin und Erlotinib umfasst.

2. *In vitro*-Verfahren zur Prognose der Reaktion oder Sensibilität auf das Hinzufügen von Bevacizumab zu einem Chemotherapie-Behandlungsplan eines Individuums, bei dem der Verdacht besteht, an Pankreaskrebs zu leiden, das an Pankreaskrebs leidet, oder das eine Disposition hat, an Pankreaskrebs zu leiden, umfassend das Bestimmen des Proteinexpressionsspiegels eines oder mehrerer von VEGFA, VEGFR2 und PLGF in einer Blutplasmaprobe eines Individuums, wobei der Pankreaskrebs metastatischer Pankreaskrebs ist und der Chemotherapie-Behandlungsplan Gemcitabin und Erlotinib umfasst.

**3.** Verfahren nach Anspruch 1, wobei der Therapieeffekt progressionsfreies Überleben ist.

**4.** Verfahren nach Anspruch 1, wobei der Therapieeffekt Gesamtüberleben ist.

**5.** Verfahren nach den Ansprüchen 2 bis 4, wobei der Proteinexpressionsspiegel, der bestimmt wird, der von VEGFA oder PLGF ist.

**6.** Verfahren nach den Ansprüchen 2 bis 4, wobei der Proteinexpressionsspiegel, der bestimmt wird, der von VEGFA oder VEGFR2 ist.

**7.** Verfahren nach den Ansprüchen 1 bis 4, wobei der Proteinexpressionsspiegel, der bestimmt wird, ein kombinierter Expressionsspiegel von VEGFA und VEGFR2 ist.

**8.** Verfahren nach den Ansprüchen 1 bis 4, wobei der Proteinexpressionsspiegel, der bestimmt wird, ein kombinierter Expressionsspiegel von VEGFA und PLGF ist.

**9.** Verfahren nach den Ansprüchen 1 bis 4, wobei der Proteinexpressionsspiegel, der bestimmt wird, ein kombinierter Expressionsspiegel von VEGFA, VEGFR2 und PLGF ist.

**10.** Verfahren nach den Ansprüchen 1 bis 9, wobei der Expressionsspiegel durch ein Immunassay-Verfahren nachgewiesen wird.

**11.** Verfahren nach Anspruch 10, wobei das Immunassay-Verfahren ELISA ist.

**12.** Verfahren nach den Ansprüchen 1 bis 11, wobei das Individuum mit einer oder mehreren Anti-Krebstherapien cotherapiert wird.

**13.** Verfahren nach Anspruch 12, wobei die Anti-Krebstherapie Bestrahlung ist.

**14.** Verfahren nach den Ansprüchen 1 bis 13, wobei die Probe vor einer neoadjuvanten oder adjuvanten Therapie oder nach einer neoadjuvanten oder adjuvanten Therapie erhalten wird.


**Revendications**

**1.** *Procédé in vitro* d'identification d'un patient réagissant à ou sensible à l'ajout d'un traitement par bévacizumab à un régime de chimiothérapie, ledit procédé comprenant la détermination du niveau d'expression protéique d'au moins l'un du VEGFA, du VEGFR2 et du PLGF dans un échantillon de plasma sanguin d'un patient soupçonné de souffrir ou susceptible de souffrir d'un cancer du pancréas, un niveau d'expression accru d'au moins l'un du VEGFA, du VEGFR2 et du PLGF relativement à des niveaux d'expression témoins déterminés chez des patients souffrant d'un cancer du pancréas indiquant une sensibilité du patient à l'ajout de bévacizumab au dit régime de chimiothérapie, dans lequel le cancer du pancréas est un cancer métastatique du pancréas et le régime de chimiothérapie comprend de la gemcitabine et de l'erlotinib.

**2.** *Procédé in vitro* de prédiction de la réponse ou de la sensibilité à l'ajout de bévacizumab à un régime de chimiothérapie d'un patient soupçonné de souffrir, souffrant ou susceptible de souffrir d'un cancer du pancréas comprenant la détermination du niveau d'expression protéique d'au moins l'un du VEGFA, du VEGFR2 et du PLGF dans un échantillon de plasma sanguin du patient, dans lequel le cancer du pancréas est un cancer métastatique du pancréas et le régime de chimiothérapie comprend de la gemcitabine et de l'erlotinib.

**3.** Procédé selon la revendication 1, dans lequel l'effet du traitement est la survie sans progression.

**4.** Procédé selon la revendication 1, dans lequel l'effet du traitement est la survie globale.

**5.** Procédé selon les revendications 2 à 4, dans lequel le niveau d'expression protéique déterminé est celui du VEGFA ou du PLGF.

**6.** Procédé selon les revendications 2 à 4, dans lequel le niveau d'expression protéique déterminé est celui du VEGFA ou du VEGFR2.

**7.** Procédé selon les revendications 1 à 4, dans lequel le niveau d'expression protéique déterminé est un niveau d'expression combinée du VEGFA et du VEGFR2.

**8.** Procédé selon les revendications 1 à 4, dans lequel le niveau d'expression protéique déterminé est un niveau d'expression combinée du VEGFA et du PLGF.

**9.** Procédé selon les revendications 1 à 4, dans lequel le niveau d'expression protéique déterminé est un niveau d'expression combinée du VEGFA, du VEGFR2 et du PLGF.

**10.** Procédé selon les revendications 1 à 9, dans lequel ledit niveau d'expression est détecté par un procédé de dosage immunologique.

**11.** Procédé selon la revendication 10, dans lequel ledit dosage immunologique est un ELISA.

**12.** Procédé selon les revendications 1 à 11, dans lequel ledit patient est co-traité avec au moins une thérapie anti-cancéreuse.

**13.** Procédé selon la revendication 12, dans lequel ladite thérapie anti-cancéreuse est une radiothérapie.

**14.** Procédé selon les revendications 1 à 13, dans lequel ledit échantillon est obtenu avant la thérapie néoadjuvante ou adjuvante ou après la thérapie néoadjuvante ou adjuvante.

**Figure 1A**

**Figure 1B**

| | bevacizumab/GE | placebo/GE |
|---|---|---|
| % events | 94.9 | 100.0 |
| Median Time (d) | 150 | 109 |
| HR | 0.649 | |
| (95% CI) | (0.495 , 0.851) | |
| Log-Rank p | 0.0017 | |

Time to First Inv PD or Death (days)

bevacizumab/GE          placebo/GE

**Figure 2A**

Bevacizumab effect in patients with low VEGFA
Overall Survival

| | |
|---|---|
| —— | Bevacizumab, n=57/52, med=214 |
| – – | Placebo, n=54/50, med=207 |

survival probability

Study Day

Bevacizumab effect in patients with high VEGFA
Overall Survival

| | |
|---|---|
| —— | Bevacizumab, n=60/50, med=226 |
| – – | Placebo, n=51/50, med=145 |

survival probability

Study Day

**Figure 2B**

Figure 3

Bevacizumab effect in patients with low VEGFR2
Overall Survival

Bevacizumab, n=56/52, med=148
Placebo, n=57/53, med=150

Bevacizumab effect in patients with high VEGFR2
Overall Survival

Bevacizumab, n=61/50, med=309
Placebo, n=50/49, med=204

**Figure 4**

Bevacizumab effect in patients with low PLGF (optimized)
Progression Free Survival

Bevacizumab, n=59/57, med=148
Placebo, n=35/35, med=161

survival probability — Study Day

Bevacizumab effect in patients with high PLGF (optimized)
Progression Free Survival

Bevacizumab, n=58/54, med=161
Placebo, n=72/72, med=101

survival probability — Study Day

**Figure 5A**

Bevacizumab effect in patients with low VEGFA & VEGFR2
Overall Survival

— bevacizumab, n=55/53, med=161
- - placebo, n=57/52, med=164

Bevacizumab effect in patients with high VEGFA & VEGFR2
Overall Survival

— bevacizumab, n=61/48, med=347
- - placebo, n=48/48, med=161

**Figure 5B**

Bevacizumab effect in patients with low VEGFA & PLGF
Overall Survival

bevacizumab, n=64/56, med=210
placebo, n=47/43, med=222

Bevacizumab effect in patients with high VEGFA & PLGF
Overall Survival

bevacizumab, n=52/45, med=275
placebo, n=58/57, med=137

**Figure 6A**

Bevacizumab effect in patients with low VEGFA & VEGFR2
Progression Free Survival

bevacizumab, n=55/55, med=129
placebo, n=57/57, med=108

Survival Probability

Study Day

Bevacizumab effect in patients with high VEGFA & VEGFR2
Progression Free Survival

bevacizumab, n=61/55, med=172
placebo, n=48/48, med=106

Survival Probability

Study Day

**Figure 6B**

Bevacizumab effect in patients with low VEGFA & PLGF
Progression Free Survival

bevacizumab, n=64/63, med=148
placebo, n=47/47, med=166

Bevacizumab effect in patients with high VEGFA & PLGF
Progression Free Survival

bevacizumab, n=52/47, med=162
placebo, n=58/58, med=75

**Figure 7A**

## Bevacizumab effect in patients with low VEGFA & VEGFR2 & PLGF
## Overall Survival

Legend:
— Bevacizumab, n=45/44, med=208
-- -- Placebo, n=44/40, med=204

## Bevacizumab effect in patients with high VEGFA & VEGFR2 & PLGF
## Overall Survival

Legend:
— Bevacizumab, n=71/57, med=235
-- -- Placebo, n=61/60, med=154

**Figure 7B**

Bevacizumab effect in patients with low VEGFA & VEGFR2 & PLGF
Progression Free Survival

Bevacizumab, n=45/45, med=133
Placebo, n=44/44, med=117

Bevacizumab effect in patients with high VEGFA & VEGFR2 & PLGF
Progression Free Survival

Bevacizumab, n=71/65, med=167
Placebo, n=61/61, med=101

**Figure 8:** SEQ ID NO:1, Exemplary amino acid sequence of VEGFA.

```
         10         20         30         40         50         60
MNFLLSWVHW SLALLLYLHH AKWSQAAPMA EGGGQNHHEV VKFMDVYQRS YCHPIETLVD

         70         80         90        100        110        120
IFQEYPDEIE YIFKPSCVPL MRCGGCCNDE GLECVPTEES NITMQIMRIK PHQGQHIGEM

        130        140        150        160        170        180
SFLQHNKCEC RPKKDRARQE KKSVRGKGKG QKRKRKKSRY KSWSVYVGAR CCLMPWSLPG

        190        200        210        220        230
PHPCGPCSER RKHLFVQDPQ TCKCSCKNTD SRCKARQLEL NERTCRCDKP RR
```

**Figure 9**: SEQ ID NO:2, Exemplary amino acid sequence of VEGFR2.

```
          10         20         30         40         50         60
MQSKVLLAVA LWLCVETRAA SVGLPSVSLD LPRLSIQKDI LTIKANTTLQ ITCRGQRDLD

          70         80         90        100        110        120
WLWPNNQSGS EQRVEVTECS DGLFCKTLTI PKVIGNDTGA YKCFYRETDL ASVIYVYVQD

         130        140        150        160        170        180
YRSPFIASVS DQHGVVYITE NKNKTVVIPC LGSISNLNVS LCARYPEKRF VPDGNRISWD

         190        200        210        220        230        240
SKKGFTIPSY MISYAGMVFC EAKINDESYQ SIMYIVVVVG YRIYDVVLSP SHGIELSVGE

         250        260        270        280        290        300
KLVLNCTART ELNVGIDFNW EYPSSKHQHK KLVNRDLKTQ SGSEMKKFLS TLTIDGVTRS

         310        320        330        340        350        360
DQGLYTCAAS SGLMTKKNST FVRVHEKPFV AFGSGMESLV EATVGERVRI PAKYLGYPPP

         370        380        390        400        410        420
EIKWYKNGIP LESNHTIKAG HVLTIMEVSE RDTGNYTVIL TNPISKEKQS HVVSLVVYVP

         430        440        450        460        470        480
PQIGEKSLIS PVDSYQYGTT QTLTCTVYAI PPPHHIHWYW QLEEECANEP SQAVSVTNPY

         490        500        510        520        530        540
PCEEWRSVED FQGGNKIEVN KNQFALIEGK NKTVSTLVIQ AANVSALYKC EAVNKVGRGE

         550        560        570        580        590        600
RVISFHVTRG PEITLQPDMQ PTEQESVSLW CTADRSTFEN LTWYKLGPQP LPIHVGELPT

         610        620        630        640        650        660
PVCKNLDTLW KLNATMFSNS TNDILIMELK NASLQDQGDY VCLAQDRKTK KRHCVVRQLT

         670        680        690        700        710        720
VLERVAPTIT GNLENQTTSI GESIEVSCTA SGNPPPQIMW FKDNETLVED SGIVLKDGNR
```

**Figure 9 (continued):** SEQ ID NO:2, Exemplary amino acid sequence of VEGFR2.

```
       730        740        750        760        770        780
NLTIRRVRKE DEGLYTCQAC SVLGCAKVEA FFIIEGAQEK TNLEIIILVG TAVIAMFFWL


       790        800        810        820        830        840
LLVIILRTVK RANGGELKTG YLSIVMDPDE LPLDEHCERL PYDASKWEFP RDRLKLGKPL


       850        860        870        880        890        900
GRGAFGQVIE ADAFGIDKTA TCRTVAVKML KEGATHSEHR ALMSELKILI HIGHHLNVVN


       910        920        930        940        950        960
LLGACTKPGG PLMVIVEFCK FGNLSTYLRS KRNEFVPYKT KGARFRQGKD YVGAIPVDLK


       970        980        990       1000       1010       1020
RRLDSITSSQ SSASSGFVEE KSLSDVEEEE APEDLYKDFL TLEHLICYSF QVAKGMEFLA


      1030       1040       1050       1060       1070       1080
SRKCIHRDLA ARNILLSEKN VVKICDFGLA RDIYKDPDYV RKGDARLPLK WMAPETIFDR


      1090       1100       1110       1120       1130       1140
VYTIQSDVWS FGVLLWEIFS LGASPYPGVK IDEEFCRRLK EGTRMRAPDY TTPEMYQTML


      1150       1160       1170       1180       1190       1200
DCWHGEPSQR PTFSELVEHL GNLLQANAQQ DGKDYIVLPI SETLSMEEDS GLSLPTSPVS


      1210       1220       1230       1240       1250       1260
CMEEEEVCDP KFHYDNTAGI SQYLQNSKRK SRPVSVKTFE DIPLEEPEVK VIPDDNQTDS


      1270       1280       1290       1300       1310       1320
GMVLASEELK TLEDRTKLSP SFGGMVPSKS RESVASEGSN QTSGYQSGYH SDDTDTTVYS


      1330       1340       1350
SEEAELLKLI EIGVQTGSTA QILQPDSGTT LSSPPV
```

**Figure 10:** SEQ ID NO:3, Exemplary amino acid sequence of PLGF.

```
         10         20         30         40         50         60
MPVMRLFPCF LQLLAGLALP AVPPQQWALS AGNGSSEVEV VPFQEVWGRS YCRALERLVD


         70         80         90        100        110        120
VVSEYPSEVE HMFSPSCVSL LRCTGCCGDE NLHCVPVETA NVTMQLLKIR SGDRPSYVEL


        130        140        150        160        170        180
TFSQHVRCEC RHSPGRQSPD MPGDFRADAP SFLPPRRSLP MLFRMEWGCA LTGSQSAVWP


        190        200        210        220
SSPVPEEIPR MHPGRNGKKQ QRKPLREKMK PERCGDAVPR R
```

Figure 11:

**Figure 12:**

Figure 13:

Figure 14:

**EP 2 596 364 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0939319 A

- EP 1610129 A

### Non-patent literature cited in the description

- **SALTZ et al.** *J. Clin. Oncol,* 2008, vol. 26, 2013-2019
- **YANG et al.** *Clin. Cancer Res.,* 2008, vol. 14, 5893-5899
- **HURWITZ et al.** *N. Engl. J. Med.,* 2004, vol. 350, 2335-2342
- **FERRARA.** *Mol. Biol. Cell,* 2010, vol. 21, 687
- **LEUNG et al.** *Science,* 1989, vol. 246, 1306
- **HOUCK et al.** *Mol. Endocrin.,* 1991, vol. 5, 1806
- **KIM et al.** *Growth Factors,* 1992, vol. 7 (1), 53-64
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **RUCZINSKI, I. et al.** *J. of Computational and Graphical Statistics,* 2003, vol. 12, 475-511
- **FRIEDMAN, J. H.** *J. of the American Statistical Association,* 1989, vol. 84, 165-175
- **HASTIE ; TREVOR ; TIBSHIRANI ; ROBERT ; FRIEDMAN ; JEROME.** The Elements of Statistical Learning. Springer Series in Statistics, 2001
- **BREIMAN, L. ; FRIEDMAN, J. H. ; OLSHEN, R. A. ; STONE, C. J.** *Classification and regression trees, California: Wadsworth,* 1984

- **BREIMAN, L.** Random Forests. *Machine Learning,* 2001, vol. 45, 5-32
- **PEPE, M. S.** The Statistical Evaluation of Medical Tests for Classification and Prediction. *Oxford Statistical Science Series,* 2003, 28
- **DUDA, R. O. ; HART, P. E. ; STORK, D. G.** Pattern Classification. Wiley Interscience, 2001
- **KOHLER et al.** Hybridoma Techniques. Cold Spring Harbor Laboratory, 1980
- **TIJSSEN.** Practice and Theory of Enzyme Immunoassays. Elsevier, 1985
- **CAMPBELL.** Monoclonal Antibody Technology. Elsevier, 1984
- **HURRELL.** Monoclonal Hybridoma Antibodies: Techniques and Applications. CRC Press, 1982
- **ZOLA.** Monoclonal Antibodies: A Manual of Techniques. CRC Press, Inc, 1987, 147-1 58
- **FERRARA, N.** *Mol. Biol. of the Cell,* 2010, vol. 21, 687-690
- **EISENHAUSER et al.** New response evaluation criteria in solid tumours: Revised RECIST guideline (version 1.1). *Eur. J. Cancer,* 2009, vol. 45, 228-247
- **LOTTSPEICH.** Bioanalytik. Spektrum Akademisher Verlag, 1998
- **SAMBROOK ; RUSSELL.** Molecular Cloning: A Laboratory Manual. CSH Press, 2001
- **VAN CUTSEM.** *J Clin. Oncol.,* 2009, vol. 27, 2231-2237

**70**